# EUROPÄISCHE PATENTSCHRIFT

(11) **EP 1 485 363 B1**
(45) Veröffentlichungstag und Bekanntmachung des Hinweises auf die Patenterteilung: **08.10.2008**
(21) Anmeldenummer: 03708178.3
(22) Anmeldetag: 05.03.2003
(51) Int. Cl.: C07D 263/58, A61K 31/445, A61P 25/00, A61P 25/16, C07D 498/04

(54) **CYCLISCHE AMIDE**
CYCLIC AMIDES
AMIDE CYCLIQUE

(30) Priorität: 12.03.2002 DE 10210779
(43) Veröffentlichungstag der Anmeldung: 15.12.2004
(73) Patentinhaber: Merck Patent GmbH, 64293 Darmstadt (DE)
(72) Erfinder: SCHADT, Oliver, 63517 Rodenbach (DE); LEIBROCK, Joachim, 64319 Pfungstadt (DE); PRUECHER, Helmut, 64646 Heppenheim (DE); SEYFRIED, Christoph, 64342 Seeheim-Jugenheim (DE)
(86) Internationale Anmeldenummer: PCT/EP2003/002224
(87) Internationale Veröffentlichungsnummer: WO 2003/076420

(56) Entgegenhaltungen:
- EP-A- 0 249 407
- EP-A- 0 360 566
- EP-A- 0 385 664
- EP-A- 0 709 384
- WO-A-01/92239
- WO-A-01/94321
- SHANKLIN, J ET AL: "diarylmethyl-aryloxymethylpiperidine derivatives and structurally related compounds" J MED CHEM, Bd. 34, Nr. 10, - 1991 Seiten 3011-3022, XP002241320
- ETSUO OHSHIMA ET AL: "Dibenzoxepine derivatives as antiallergic agents" J MED CHEM, Bd. 36, Nr. 3, - 1993 Seiten 417-420, XP002241321

## Beschreibung

Die Erfindung betrifft Verbindungen der Formel I 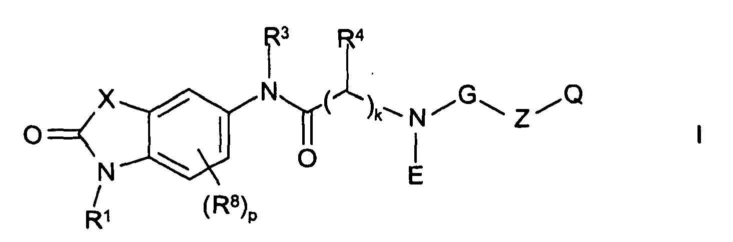 worin
- R¹: H oder A
- A: geradkettiges oder verzweigtes Alkyl mit 1 bis 10 C-Atomen, Alkenyl mit 2 bis 10 C-Atomen, Alkoxy mit 1 bis 10 C-Atomen oder Alkoxyalkyl mit 2 bis 10 C-Atomen,
- X: O, S, N-R², CH₂ oder CH₂CH₂,
- R²: H oder A und
- R³: H oder A bedeutet,
- R⁴: unabhängig ausgewählt ist unter H, A oder (CH₂)ₙAr,
- R⁶: für H, A oder Cycloalkyl mit 3 bis 7 C-Atomen, und
- R⁸: A, Hal, CN, NO₂, NH₂, CF₃, OCF₃ oder SO₂CH₃, und
- k: für 1 oder 2 steht,
die Gruppe 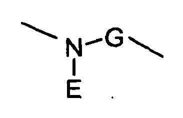 ausgewählt ist unter den Gruppen 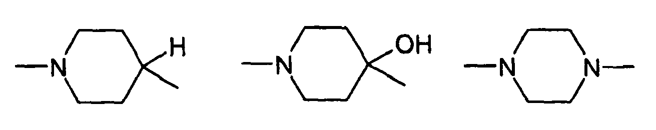 und 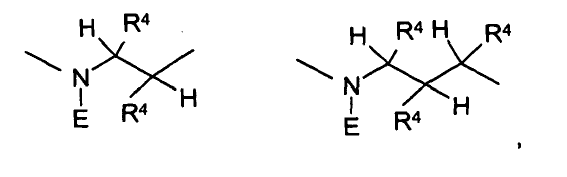
- E: H, A, (CH₂)ₙHet, (CH₂)ₙAr oder Cycloalkyl mit 3 bis 7 C-Atomen bedeutet,
- Z: O, S, N-R¹⁵, CH₂ oder CH₂CH₂,
- R¹⁵: H, A, (CH₂)ₙHet, (CH₂)ₙAr oder Cycloalkyl mit 3 bis 7 C-Atomen,
- Het: einen unsubstituierten oder einfach oder mehrfach durch A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A und/oder OOCR⁶ substituierten, gesättigten, ungesättigten oder aromatischen mono- oder bicyclischen heterocyclischen Rest, ausgewählt unter 1-Piperidyl, 1-Piperazyl, 1-(4-Methyl)-piperazyl, 4-Methylpiperazin-1-ylamin, 4-Morpholinyl, 1-Pyrrolidinyl, 1-Pyrazolidinyl 1-(2-Methyl)-pyrazolidinyl, 1-Imidazolidinyl oder 1-(3-Methyl)-imidazolidinyl,Thiophen-2-yl oder Thiophen-3-yl, 2-, 3- oder 4-Pyridyl, das unsubstituiert oder durch eine oder mehrere CN-Gruppe substituiert sein kann, 2-, 4- oder 5-Oxazolyl, 2-, 4- oder 5-Thiazolyl, Chinolinyl, Isochinolinyl, 2- oder 4-Pyridazyl, 2-, 4- oder 5-Pyrimidyl, 2- oder 3-Pyrazinyl,
- Ar: einen unsubstituierten oder einfach oder mehrfach durch A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A und/oder OOCR⁶ substituierten aromatischen Kohlenwasserstofferest mit 6 bis 14 Kohlenstoffatomen,
- n: 0, 1, 2, 3, 4 oder 5,
- p: 0, 1, 2 oder 3,
- w: 0, 1, 2 oder 3,
und
- Hal: F, Cl, Br oder I
bedeuten, und die Gruppe
Z-Q ausgewählt ist unter den Gruppen 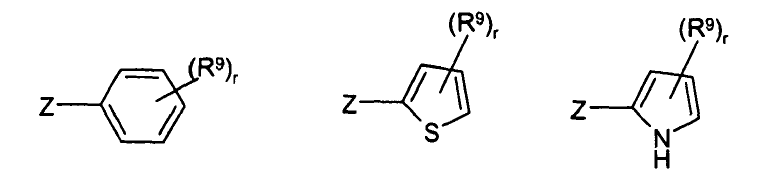 worin
- R⁹: unabhängig ausgewählt ist unter Hal und A,
- r: für 0, 1, 2, 3, 4 oder 5 steht,
sowie die pharmazeutisch verwendbaren Derivate, Salze, Solvate und Stereoisomere einschließlich deren Mischungen in allen Verhältnissen, und insbesondere deren Salze und Solvate.

Benzylpiperidinderivate mit hoher Affinität zu Bindungsstellen von Aminosäure-Rezeptoren sind z.B. aus der EP 0 709 384 A1 bekannt.

Der Erfindung lag die Aufgabe zugrunde, neue Verbindungen mit wertvollen Eigenschaften aufzufinden, insbesondere solche, die ein verbessertes Wirkprofil aufweisen, zum Beispiel eine höhere Wirksamkeit, eine höhere Selektivität oder ein breiteres Anwendungsprofil und/oder geringere Nebenwirkungen. Die neuen Verbindungen sollen vorzugsweise einfach und kostengünstig herstellbar sein und sich insbesondere zur Herstellung von Arzneimitteln eignen.

Überraschend wurde gefunden, dass die Aufgabe durch die Verbindungen der Formel I gelöst wird. Insbesondere wurde gefunden, dass die Verbindungen der Formel I und ihre Salze bei guter Verträglichkeit sehr wertvolle pharmakologische Eigenschaften besitzen. Vor allem zeigen sie eine besonders hohe Affinität zu Bindungsstellen von Aminosäure-Rezeptoren, insbesondere zur Ifenprodil-Bindungsstelle am NMDA-Rezeptor (NMDA = N-Methyl-D-aspartat), die die Polyamin-Bindungsstelle allosterisch moduliert. Vorzugsweise sind die erfindungsgemäßen Verbindungen Liganden der Ifenprodil-Bindungsstelle des NMDA-Rezeptors und gehören somit zum Gebiet der NR2B-Antagonisten. Besonders bevorzugt sind die erfindungsgemäßen Verbindungen Liganden der Ifenprodil-Bindungsstelle des NMDA-2B-Rezeptors.

Der Bindungstest für [³H]-Ifenprodil kann nach der Methode von Schoemaker et al., Eur. J. Pharmacol. 176, 249-250 (1990) durchgeführt werden. Die Verbindungen eignen sich zur Behandlung von neurodegenerativen Erkrankungen einschließlich cerebrovaskulären Krankheiten. Ebenso können die neuen Verbindungen als Analgetikum oder Anxiolytikum sowie zur Behandlung von Epilepsie, Schizophrenie, der Alzheimer-, der Parkinson- bzw. der Huntington-Krankheit, cerebralen Ischämien oder Infarkten verwendet werden. Ferner eignen sie sich zur Behandlung von Psychosen, bedingt durch überhöhte Aminosäurespiegel.

Der [³H]-CGP-39653-Bindungstest für die Glutamat-Bindungsstelle des NMDA-Rezeptors kann beispielsweise nach der Methode von M.A.Stills et al., beschrieben in Eur. J. Pharmacol. 192, 19-24 (1991), durchgeführt werden. Der Test für die Glycin-Bindungsstelle des NMDA-Rezeptors ist durchführbar nach der Methode von M.B. Baron et al., beschrieben in Eur. J. Pharmacol. 206, 149-154 (1991).

Die Wirkung gegen Morbus Parkinson, d.h. die Potenzierung des L-DOPAinduzierten kontralateralen Drehens bei hemiparkinsonischen Ratten, ist nach der Methode von U. Ungerstedt und G.W. Arbuthnott, Brain Res. 24, 485 (1970) nachweisbar.

Besonders geeignet ist die Verbindung zur Behandlung oder Prophylaxe von Schlaganfällen sowie zum Schutz vor und zur Behandlung von Hirnödemen und Unterversorgungszuständen des Zentralnervensystems, vor allem Hypoxie oder Anoxie.

Die genannten Wirkungen können außerdem nach den Methoden nachgewiesen oder überprüft werden, wie sie in den folgenden Literaturstellen beschrieben sind:
J.W. McDonald, F.S. Silverstein und M.V. Johnston, Eur. J. Pharmacol. 140, 359 (1987); R. Gill, A.C. Foster und G.N. Woodruff, J. Neurosci. 7, 3343 (1987); J.B. Bederson et al., Stroke, 17, 472-476 (1986); S. Brint et al., J. Cereb. Blood Flow Metab. 8, 474-485 (1988).

Aus den nachfolgend aufgeführten Literaturstellen sind verschiedene Antagonisten bekannt, die verschiedene Bindungsstellen des NMDA-Rezeptors blockieren können:
W. Danysz, C.G. Parsons, I. Bresink und G. Quack, Drug, News & Perspectives 8, 261 (1995), K.R. Gee, Exp. Opin. Invest. Drugs 3, 1021 (1994) und J.J. Kulagowski und L.L. Iversen, J. Med. Chem. 37, 4053 (1994).

Ifenprodil und Eliprodil der Formeln III bzw. IV können den NMDA-Rezeptor blockieren, indem sie eine Wechselwirkung mit der modulatorischen Polyamin-Bindungsstelle eingehen (C.J. Carter, K.G.Lloyd, B. Zivkovic und B. Scatton, J. Pharmacol. Exp. Ther. 253, 475 (1990)).

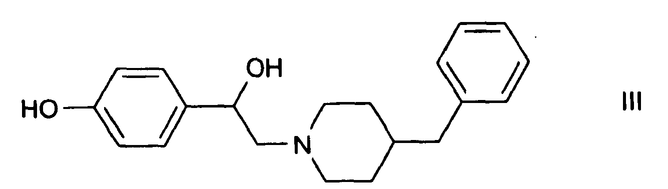

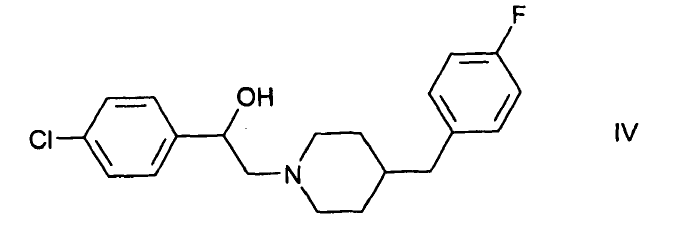

Da Ifenprodil und Eliprodil mit der Polyamin-Bindungsstelle am NMDA-Rezeptor wechselwirken, kann die antagonistische Aktivität der erfindungsgemäßen Verbindungen in einem Spermin-stimulierten [³H]MK-801 (Dizocilpine) - Bindungstest ermittelt werden.
In der Gegenwart von Sättigungskonzentrationen von Glycin und NMDA, kann Spermin noch die Bindung von MK-801 erhöhen, die durch Ifenprodil, Eliprodil und ganz besonders wirksam durch die erfindungsgemäßen Verbindungen inhibiert wird.

Zusätzlich können die erfindungsgemäßen Verbindungen in einem [³H]GABA (y-Aminobuttersäure) - Freisetzungstest, analog J. Dreijer, T. Honoré und A. Schousboe, J. Neurosci. 7, 2910 (1987), der als in-vitro-Modell die antagonistische Funktion in der Zelle beschreibt, getestet werden.

Gegenstand der Erfindung sind demgemäss die Verbindungen der Formel I nach Anspruch 1 und/oder deren pharmazeutisch verwendbaren Derivate, Solvate und Stereoisomere, einschließlich deren Mischungen in allen Verhältnissen und insbesondere deren physiologisch unbedenkliche Salze als Antagonisten an Rezeptoren von exzitatorischen Aminosäuren, wie z.B. Glutaminsäure bzw. deren Salze.

Gegenstand der Erfindung sind die Verbindungen der Formel I nach Anspruch 1 und/oder deren pharmazeutisch verwendbaren Derivate, Solvate und Stereoisomere, einschließlich deren Mischungen in allen Verhältnissen und insbesondere deren physiologisch unbedenkliche Salze als Glycin-Transporter-Inhibitor.

Gegenstand der Erfindung sind insbesondere die Verbindungen der Formel I nach Anspruch 1 und/oder deren pharmazeutisch verwendbaren Derivate, Solvate und Stereoisomere, einschließlich deren Mischungen in allen Verhältnissen und insbesondere deren physiologisch unbedenkliche Salze als exzitatorische Aminosäuren-Antagonisten zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen.

Gegenstand der Erfindung ist auch die Verwendung der Verbindungen der Formel I nach Anspruch 1 und/oder deren pharmazeutisch verwendbaren Derivate, Solvate und Stereoisomere, einschließlich deren Mischungen in allen Verhältnissen und insbesondere deren physiologisch unbedenkliche Salze zur Herstellung eines Arzneimittels zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen.

In diesem Zusammenhang wird auf die WO00/00197 verwiesen, auf deren Offenbarung hiermit in vollem Umfang Bezug genommen wird.

Die Verbindungen der Formel I können als Arzneimittelwirkstoff in der Human- und Veterinärmedizin eingesetzt werden.

Gegenstand der Erfindung ist ferner ein Verfahren zur Herstellung der Verbindungen der Formel I nach Anspruch 1 sowie ihrer physiologisch unbedenklichen Salze, dadurch gekennzeichnet, dass man
a) eine Verbindung der Formel II 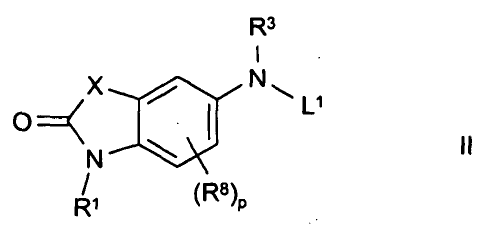 worin
   - L¹: H oder ein Metallion bedeutet,
   und R¹, X, R³, R⁸ und p die vorstehend und nachstehend angegebenen Bedeutungen haben,
b) mit einer Verbindung der Formel V 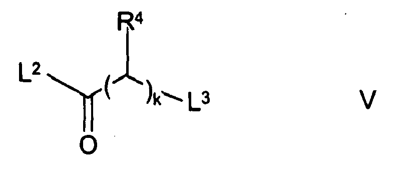 worin
   - L²: Cl, Br, I, OH oder eine reaktionsfähig veresterte OH- Gruppe bedeutet,
   - L³: Cl, Br, I, OH oder eine Diazoniumgruppe bedeutet und R⁴ und k die vorstehend und nachstehend angegebenen Bedeutungen haben,
   und
c) mit einer Verbindung der Formel VI 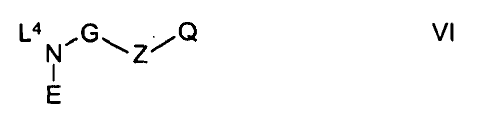 worin
   - L4: H oder ein Metallion bedeutet und E, G, Z, und Q die vorstehend und nachstehend angegebenen Bedeutungen haben,
   umsetzt, und gegebenenfalls
d) die erhaltene Verbindung der Formel I durch Behandeln mit einer Säure in eines ihrer Salze überführt.

In einer bevorzugten Ausführungsform ist die Verbindung der Formel VI ausgewählt unter Verbindungen der Formeln 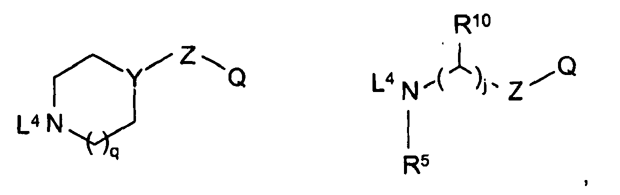 worin L⁴, q, Y, R⁵, R¹⁰, Z, j und Q wie vorstehend und nachstehend definiert sind.

Vorzugsweise ist die Verbindung der Formel VI ausgewählt unter Verbindungen der Formeln 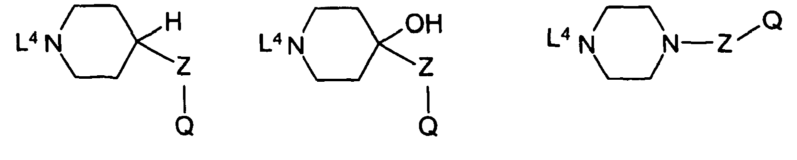 und 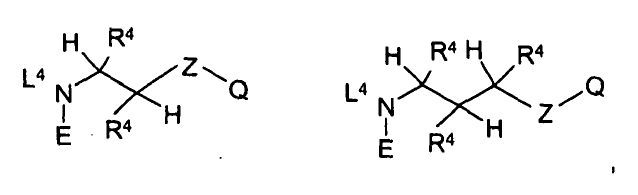 worin L⁴, R⁴, Z, Q und E wie vorstehend und nachstehend definiert sind.

Vorzugsweise ist die Verbindung der Formel VI ausgewählt unter Verbindungen der Formeln 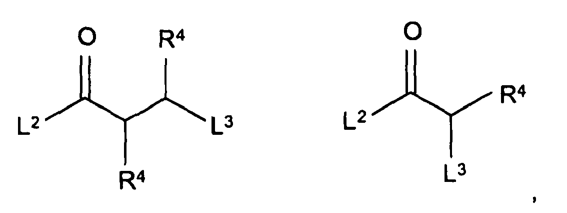 worin L², L³ und R⁴ wie vorstehend und nachstehend definiert sind.

Das erfindungsgemäße Verfahren kann im Sinne einer Eintopfreaktion durchgeführt werden, d. h. auf Isolierungs- und/oder Reinigungsschritte wird so weit wie möglich verzichtet und nur das gewünschte Endprodukt, d. h. in der Regel eine erfindungsgemäße Verbindung oder ein pharmazeutisch verwendbares Derivat davon, gereinigt und/oder isoliert. Alternativ kann nach jedem der genannten Reaktionsschritte ein Reinigungs- und/oder Isolierungsschritt durchgeführt werden. Auch gemischte Formen der vorstehend beschriebenen Verfahrensweisen sind denkbar. Geeignete Reinigungs- und Isolierungsschritte sind dem Fachmann bekannt, z. B. aus Houben-Weyl, Methoden der organischen Chemie, Georg-Thieme-Verlag, Stuttgart.

Gegenstand der Erfindung sind insbesondere diejenigen Verbindungen der Formel I, in denen mindestens einer der genannten Reste eine der vorstehend oder nachstehend angegebenen bevorzugten Bedeutungen hat.

Im Rahmen der vorliegenden Erfindung bedeutet Alkyl einen linearen oder verzweigten Alkylrest, vorzugsweise einen unverzweigten Alkylrest, der 1, 2, 3, 4, 5, 6, 7, 8, 9 oder 10 C-Atome, vorzugsweise 1, 2, 3, 4 oder 5 C-Atome aufweist und ein- oder mehrfach mit Halogen (Hal), z. B. perfluoriert, sein kann. Wenn ein Alkylrest mit Halogen substituiert ist, weist er vorzugsweise, abhängig von der Anzahl der Kohlenstoffatome des Alkylrests, 1, 2, 3, 4 oder 5 Halogenatome auf. So kann beispielsweise eine Methylgruppe (Alkylrest mit 1 Kohlenstoffatom) 1-, 2- oder 3-fach mit Halogen substituiert sein, und eine Ethylgruppe (Alkylrest mit 2 Kohlenstoffatomen) 1-, 2-, 3-, 4- oder 5-fach mit Halogen substituiert sein.
Für Alkylgruppen mit mehr als 2 Kohlenstoffatomen gilt vorzugsweise das gleiche wie für Ethylgruppen. Besonders bevorzugt steht Alkyl für Methyl, Ethyl, Trifluormethyl, Pentafluorethyl oder Propyl, weiterhin bevorzugt für Isopropyl, Butyl, Isobutyl, sek.-Butyl oder tert.-Butyl, aber auch für n-Pentyl, neo-Pentyl, Isopentyl oder Hexyl.

Der Ausdruck "Alkylen" steht vorzugsweise für einen divalenten Kohlenstoffrest mit vorzugsweise 1 bis 10 Kohlenstoffatomen und insbesondere 1 bis 6 Kohlenstoffatomen, der gegebenenfalls ein oder mehrfach substituiert sein kann. Geeignete Substituenten für Alkylenreste sind beispielsweise Halogen, Hydroxy-Gruppen, Alkylreste, Alkoxyreste, Aminogruppen und Alkylaminogruppen. Bevorzugt steht Alkylen für Methylen, Ethylen, n-Propylen und n-Butylen.

Der Ausdruck "Alkenyl" umfasst vorzugsweise ein- oder mehrfach ethylenisch ungesättigte, geradkettige oder verzweigte Kohlenwasserstoffereste mit 2 bis 10 und insbesondere 3 bis 6 Kohlenstoffatomen, und insbesondere Allyl, 2- oder 3-Butenyl, Isobutenyl, sek.-Butenyl, ferner bevorzugt ist 4-Pentenyl, iso-Pentenyl oder 5-Hexenyl.

Der Ausdruck "Alkoxy" steht vorzugsweise für Reste der Formel -O-Alkyl, worin Alkyl die vorstehend genannte Bedeutung hat, oder, wenn zwei Alkoxyreste an benachbarte (vicinale) Kohlenstoffatome gebunden sind, für -O-Alkylen-O-, worin Alkylen die vorstehend genannte Bedeutung hat. Bevorzugte Alkoxyreste der Formel -O-Alkyl sind Methoxy, Ethoxy und Propoxy. Bevorzugte Alkoxyreste der Formel -O-Alkylen-O- sind -O-CH₂-O-, -O-CH₂CH₂-O- und -O-CH₂CH₂CH₂-O-.

Der Ausdruck "Alkoxyalkyl" umfasst vorzugsweise geradkettige Reste der Formel CᵤH₂ᵤ₊₁-O-(CH₂)ᵥ, worin u und v jeweils unabhängig voneinander 1 bis 6 bedeuten. Besonders bevorzugt ist u = 1 und v = 1 bis 4.

Der Ausdruck "Aryl" umfasst vorzugsweise einen unsubstituierten oder ein- oder mehrfach substituierten Benzolring, z.B. einen unsubstituierten oder substituierten Phenylrest oder ein unsubstituiertes oder ein- oder mehrfach substituiertes System aus Benzolringen, wie zum Beispiel Anthracen-, Phenanthren- oder Napthalen-Ringsysteme. Beispiele für geeignete Substituenten umfassen Alkyl-, Alkoxy-, Oxo-, Hydroxy-, Mercapto-, Amino-, Nitro-, Cyano- und Halogen-Reste.

Der Ausdruck "Aralkyl" umfasst vorzugsweise einen Arylrest wie obenstehend definiert, verbunden mit einem Alkylrest wie obenstehend definiert. Beispiele für geeignete Aralkylreste umfassen, sind aber nicht beschränkt auf, Benzyl, Phenylpropyl, Phenylbutyl und dergleichen.

Ar steht vorzugsweise für einen unsubstituierten oder ein- oder mehrfach durch A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A und/oder OOCR⁶ substituierten Arylrest und insbesondere für unsubstituiertes oder wie vorstehend substituiertes Phenyl, Naphthyl oder Biphenyl.

Het ist vorzugsweise ein unsubstituierter oder durch A und/oder Hal substituierter heteroaromatischer Rest und insbesondere ein unsubstituierter oder durch A und/oder Hal substituierter gesättigter heterocyclischer Rest. Bevorzugt bedeutet Het 1-Piperidyl, 1-Piperazyl, 1-(4-Methyl)-piperazyl, 4-Methylpiperazin-1-ylamin, 4-Morpholinyl, 1-Pyrrolidinyl, 1-Pyrazolidinyl 1-(2-Methyl)-pyrazolidinyl, 1-Imidazolidinyl oder 1-(3-Methyl)-imidazolidinyl,Thiophen-2-yl oder Thiophen-3-yl, 2-, 3- oder 4-Pyridyl, das unsubstituiert oder durch eine oder mehrere CN-Gruppe substituiert sein kann, 2-, 4- oder 5-Oxazolyl, 2-, 4- oder 5-Thiazolyl, Chinolinyl, Isochinolinyl, 2- oder 4-Pyridazyl, 2-, 4- oder 5-Pyrimidyl, 2- oder 3-Pyrazinyl.

In den Verbindungen der Formel I und den Verbindungen der Formel VI ist die Gruppe Z-Q ausgewählt unter den Gruppen 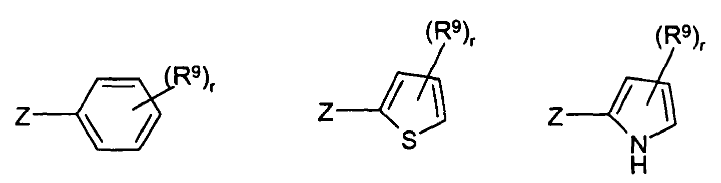 worin
- R⁹: unabhängig ausgewählt ist unter Hal und A, und
- r: für 0, 1, 2, 3, 4 oder 5 und insbesondere 0, 1, 2 oder 3 steht.

Wenn die Gruppe Z-Q für die Gruppe 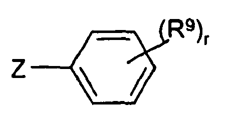 steht, ist sie vorzugsweise ausgewählt unter den Gruppen 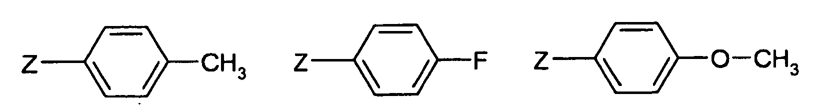 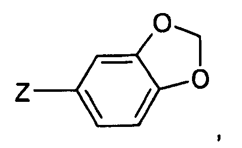 worin Z wie vorstehend definiert ist.

In den Verbindungen der Formel I ist die Gruppe 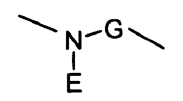 ausgewählt unter den Gruppen 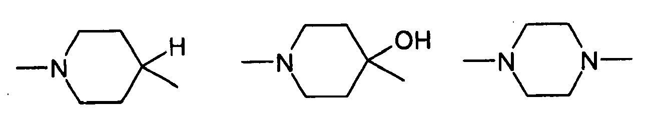 und 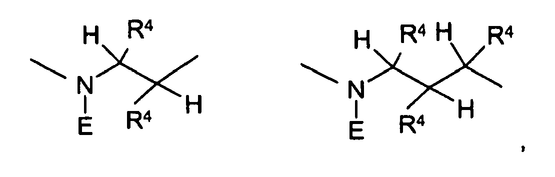 worin R⁴ und E wie vorstehend und nachstehend definiert ist.

Eine bevorzugte Ausführungsform der vorliegenden Erfindung betrifft Verbindungen der Formel I, worin R¹, X, R³, R⁸, p, R⁴, k und Z wie vorstehend und nachstehend definiert sind und E und G zusammen mit dem N-Atom, an das sie gebunden sind, für einen 5-, 6- oder 7-gliedrigen Heterocyclus stehen, der 1 oder 2 weitere Heteroatome, ausgewählt unter N, O und S, aufweisen kann, und worin Q ausgewählt ist unter substituierten oder unsubstituierten Phenylresten, vorzugsweise substituierten Phenylresten, und substituierten oder unsubstituierten Thiophen-2-yl-Resten, vorzugsweise unsubstituiertenThiophen -2-yl-Resten. In dieser Ausführungsform steht X vorzugsweise für O, R¹ und R³ vorzugsweise für H oder A, und k vorzugsweise für 1 oder 2 und insbesondere für 1. In dieser Ausführungsform steht R⁴ vorzugsweise unabhängig für Aryl, H oder A besonders bevorzugt für H, Methyl oder Phenyl und insbesondere für H.

Bevorzugte Verbindungen der Formel I sind Verbindungen der Formel Ia, 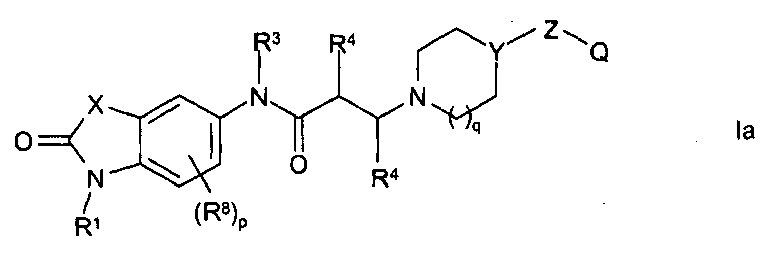 worin R¹, X, R³, R⁸, p, Z und Q wie vorstehend und nachstehend definiert sind und die beiden Reste R⁴ unabhängig voneinander ausgewählt sind unter den vorstehend und nachstehend angegebenen Bedeutungen für R⁴,
und worin
- q: 0, 1, 2 oder 3,
- Y: CH, COR⁷, oder N, und
- R⁷: H bedeuten,
sowie deren pharmazeutisch verwendbaren Derivate, Solvate und Stereoisomere, einschließlich deren Mischungen in allen Verhältnissen, und insbesondere deren Salze und Solvate.

Bevorzugte Verbindungen der Formel I sind Verbindungen der Formel Ib, 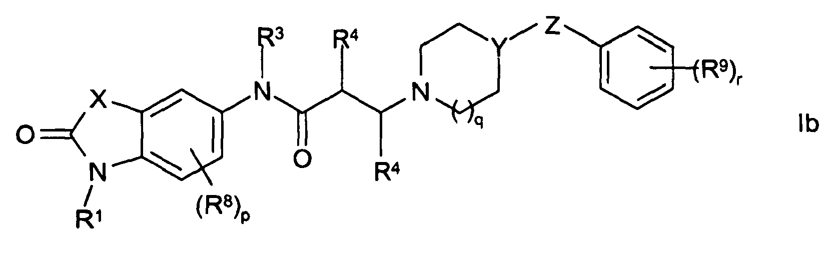 worin die Reste
- R⁹: wie vorstehend definiert sind,
- r: für 0, 1, 2, 3, 4 oder 5 steht,
und worin R¹, R², R³, R⁴, R⁸, p, q, X, Y und Z wie vorstehend und nachstehend definiert sind,
sowie deren pharmazeutisch verwendbaren Derivate, Solvate und Stereoisomere, einschließlich deren Mischungen in allen Verhältnissen, und insbesondere deren Salze und Solvate.

Gegenstand der vorliegenden Erfindung sind bevorzugt Verbindungen der Formel I, la und insbesondere Verbindungen der Formel Ib wie vorstehend beschrieben, worin R¹, R², R⁸, r, Ar und Het die vorstehend genannten Bedeutungen aufweisen, und worin
- A: geradkettiges Alkyl mit 1 bis 4 C-Atomen, verzweigtes Alkyl mit 3 bis 6 C-Atomen, Alkoxy mit 1 bis 4 Kohlenstoffatomen,
- X: O oder N-R², und
- R³: H oder A bedeutet,
- R⁴: unabhängig voneinander ausgewählt sind unter H, A und (CH₂)ₙAr,
- Y: CH, COR⁷ oder N,
- R⁷: H,
- Z: O, CH₂ oder CH₂CH₂,
- n, m: unabhängig voneinander 0, 1, 2 oder 3,
- p: 0, 1 oder 2,
- q: 1 oder 2
bedeuten, und
- R⁹: wie vorstehend definiert ist.
sowie deren Solvate und Salze.

Vorzugsweise ist die Summe aus n und m größer als Null.

Einige bevorzugte Gruppen von Verbindungen der Formeln I, Ia und/oder Ib können durch die folgenden Teilformeln Iα) bis Iζ) ausgedrückt werden, die der Formel I und/oder Ia und insbesondere der Formel Ib entsprechen und worin die nicht näher bezeichneten Reste die vorstehend angegebene Bedeutung haben, worin jedoch

| | | |
|---|---|---|
| in Iα) | R¹ | H oder Methyl bedeutet; |
| | | |
| in Iβ) | R¹ | H oder Methyl und |
| | X | O, S oder NR² und insbesondere O bedeutet; |
| | | |
| in Iγ) | R¹ | H oder Methyl, |
| | X | O, S oder NR² und insbesondere O, |
| | R² | H, |
| | R³ | H oder A und insbesondere H oder Methyl, und |
| | R⁹ | Hal bedeutet; |
| | | |
| in Iδ) | R¹ | H oder Methyl, |
| | X | O, S oder NR² und insbesondere O, |
| | R² | H, |
| | R³ | H oder A und insbesondere H oder Methyl, |
| | Y | CH, COR⁷ oder N, und |
| | R⁹ | Hal, Alkyl oder Alkoxy bedeutet; |
| | | |
| in Iε) | R¹ | H oder Methyl, |
| | X | O, S oder NR² und insbesondere O, |
| | R² | H, |
| | R³ | H oder A und insbesondere H oder Methyl, |
| | Y | CH, COR⁷ oder N und insbesondere CH, |
| | R⁷ | H, |
| | Z | O, CH₂, CH₂CH₂ oder N-R¹⁵, |
| | R¹⁵ | H oder A, |
| | R⁸ | Hal oder A und insbesondere Cl, und |
| | p | 0 oder 1 bedeutet; |
| | | |
| in Iζ) | R¹ | H oder Methyl, |
| | X | O, S oder NR² und insbesondere O, |
| | R² | H, |
| | R³ | H oder A und insbesondere H oder Methyl, |
| | Y | CH, COR⁷ oder N und insbesondere CH, |
| | R⁷ | H, |
| | Z | O, CH₂, CH₂CH₂ oder N-R¹⁵, |
| | R¹⁵ | H oder A, |
| | R⁸ | Hal oder A und insbesondere Cl, |
| | p | 0 oder 1 bedeutet, und |
| | R⁹ | Hal, Alkyl oder Alkoxy bedeutet. |

In einer Ausführungsform der vorliegenden Erfindung steht n für 0 oder 1 und insbesondere für 0.

Eine bevorzugte Ausführungsform der vorliegenden Erfindung betrifft Verbindungen, in denen ein Rest R⁴ oder beide Reste R⁴ für H stehen.

In einer bevorzugten Ausführungsform der vorliegenden Erfindung und insbesondere in den Teilformeln Iα) bis Iζ) steht der Rest R⁸ für A, Hal, insbesondere F oder Cl, CN, NO₂ NH₂, CF₃, OCF₃ oder SO₂CH₃, besonders bevorzugt für A oder Cl; und p für 0, 1 oder 2, besonders bevorzugt 0 oder 1.

In einer bevorzugten Ausführungsform der vorliegenden Erfindung steht in der Formel Ia und insbesondere in den Teilformeln Iα) bis Iε) der Rest R³⁹ für A, F, Cl, Br, I, CN, NO₂, NH₂, CF₃, OCF₃ oder SO₂CH₃.

In einer besonders bevorzugten Ausführungsform der vorliegenden Erfindung steht in der Formel Ia und insbesondere in den Teilformeln Iα) bis Iζ) der Rest R³⁹ für F und insbesondere F in 4-Stellung, für substituiertes oder vorzugsweise unsubstituiertes Alkyl mit 1 bis 4 Kohlenstoffatomen, besonders bevorzugt Alkyl wie vorstehend definiert in 4-Stellung, ganz besonders bevorzugt Methyl und insbesondere 4-Methyl, oder für Alkoxy, vorzugsweise unsubstituiertes Alkoxy mit 1 bis 3 Kohlenstoffatomen, wobei r vorzugsweise für 0, 1, 2 oder 3 und besonders bevorzugt für 1 oder 2 steht. Im Rahmen dieser Ausführungsform steht Alkoxy vorzugsweise für Methoxy, wenn nur einer der Reste R³⁹ für Alkoxy steht und insbesondere wenn R³⁹ für Alkoxy und r für 1 steht. Im Rahmen dieser Ausführungsform steht Alkoxy vorzugsweise für -O-Alkylen-O- mit 1 bis 3 Kohlenstoffatomen und insbesondere für -O-CH₂-O- oder -O-CH₂CH₂-O-, wenn zwei der Reste R³⁹ für Alkoxy stehen und insbesondere wenn zwei Reste R³⁹ für Alkoxy stehen und r für 2 steht. Im Rahmen dieser steht Ausführungsform steht einer der Reste R³⁹ vorzugsweise in 4-Stellung.

In einer bevorzugten Ausführungsform der vorliegenden Erfindung und insbesondere in den Teilformeln Iα) bis Iζ) stehen die Reste R⁴ unabhängig voneinander vorzugsweise für H, Methyl, Ethyl, Trifluormethyl, Pentafluorethyl, Propyl, Isopropyl, Butyl, Isobutyl, sek.-Butyl oder tert.-Butyl, besonders bevorzugt für H, Methyl, Ethyl, Trifluormethyl, Pentafluorethyl und insbesondere für H oder Methyl.

In einer speziellen und bevorzugten Ausführungsform betrifft die vorliegende Erfindung eine Verbindung der Formel I und insbesondere eine Verbindung der Formel Ia, worin X für O steht, sowie deren pharmazeutisch verwendbaren Derivate, Solvate, Salze, Stereoisomere und Mischungen davon und insbesondere die Solvate und Salze der Verbindungen.

In einer weiteren speziellen und bevorzugten Ausführungsform betrifft die vorliegende Erfindung eine Verbindung der Teilformeln Iα) bis Iζ) wie vorstehend definiert, worin X für O steht, deren pharmazeutisch verwendbaren Derivate, Solvate, Salze, Stereoisomere und Mischungen davon und insbesondere die Solvate und Salze der Verbindungen.

In einer weiteren bevorzugten Ausführungsform betrifft die vorliegende Erfindung eine Verbindung der Formel I, bevorzugt eine Verbindung der Formel Ia und insbesondere eine Verbindung der Teilformeln Iα) bis Iγ), worin Y für CH, CHOH (d. h. für COR⁷, worin R⁷ für H steht) oder N und insbesondere für CH steht, sowie deren Solvate und Salze.

In einer weiteren bevorzugten Ausführungsform der vorliegenden Erfindung ist in den Verbindungen der Formel Ib und insbesondere in den Teilformeln Iα) bis Iζ) die Gruppe 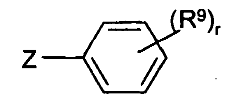 ausgewählt unter den Gruppen 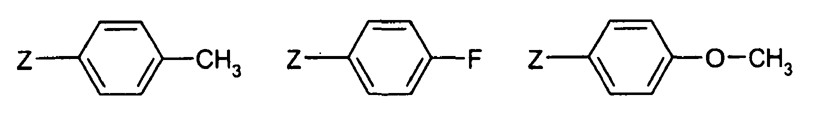 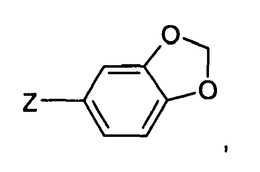 worin Z wie vorstehend definiert ist.

In einer ganz besonders bevorzugten Ausführungsform betrifft die vorliegende Erfindung eine Verbindung der Formel I, vorzugsweise der Formel Ia, besonders bevorzugt der Formel Ib und insbesondere eine Verbindung der Teilformeln Iα) bis Iζ), welche die Merkmale einer oder mehrerer der vorstehend beschriebenen Ausführungsformen und insbesondere die Merkmale aller vorstehend beschriebenen Ausführungsformen umfasst.

In einer ganz besonders bevorzugten Ausführungsform der vorliegenden Erfindung sind die Verbindungen der Formel I ausgewählt unter
a) 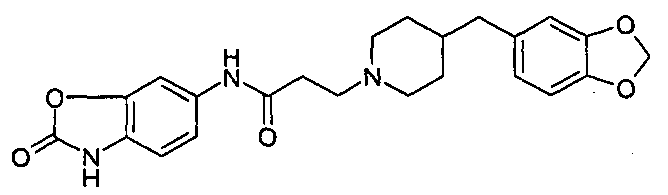 3-[4-(3,4-Methylendioxybenzyl)-piperidino]-N-(2-oxo-2,3-dihydrobenzoxazol-6-yl)-propionamid
b) 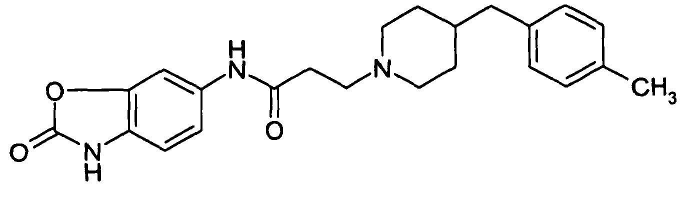 3-[4-(4-Methylbenzyl)-piperidino]-N-(2-oxo-2,3-dihydrobenzoxazol-6-yl)-propionamid
c) 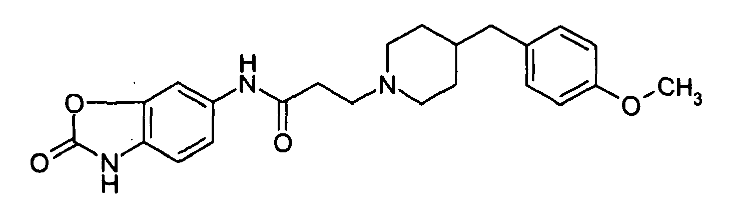 3-[4-(4-Methoxybenzyl)-1-piperidyl]-N-(2-oxo-2,3-dihydrobenzoxazol-6-yl)-propionamid
d) 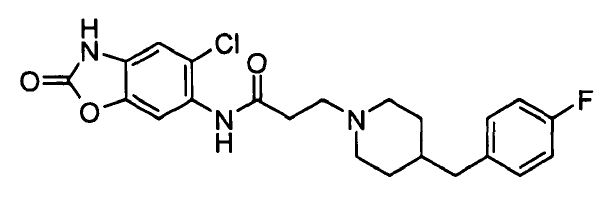 5-Chlor-6-[3-(4-(4-fluorbenzyl)-1-piperidyl)-propionamido]-2,3-dihydrobenzoxazol-2-on
e) 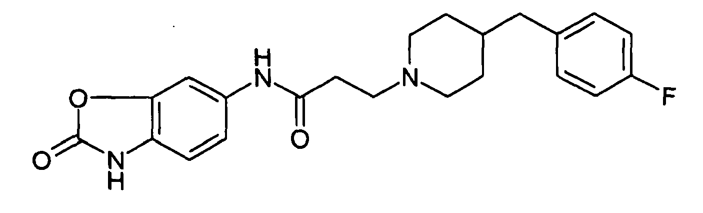 3-[4-(4-Fluorbenzyl)-1-piperidyl]-N-(2-oxo-2,3-dihydrobenzoxazol-6-yl)-propionamid
f) 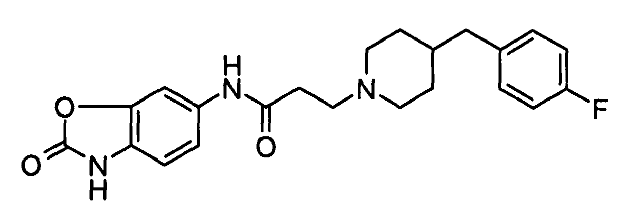 N-(2,3-Dihydro-2-oxo-6-benzoxazolyl)-3-[4-(4-fluorbenzyl)-1-piperidyl]-propionamid
sowie deren pharmazeutisch verwendbaren Derivaten, Solvaten und Stereoisomeren, einschließlich deren Mischungen in allen Verhältnissen, und insbesondere deren Salzen und Solvaten.

Weiterhin bevorzugte Verbindungen der Formel I sind Verbindungen der Formel Ic, 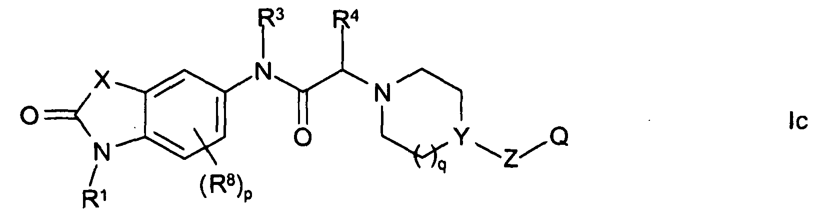 worin
- Q: die vorstehend und nachstehend angegebene Bedeutung aufweist und worin
- R¹: H oder A
- A: geradkettiges oder verzweigtes Alkyl mit 1 bis 10 C-Atomen, Alkenyl mit 2 bis 10 C-Atomen, Alkoxy mit 1 bis 10 C-Atomen oder Alkoxyalkyl mit 2 bis 10 C-Atomen,
- X: O, S, N-R², CH, oder CH₂CH₂,
- R²: H oder A,
- R³: H oder A,
- R⁴: H, A oder (CH₂)ₙAr,
- R⁶: H, A oder Cycloalkyl mit 3 bis 7 C-Atomen,
- Y: CH, COH oder N,
- Z: O, S, N-R¹⁵, CH₂ oder CH₂CH₂,
- R¹⁵: H, A, (CH₂)ₙHet, (CH₂)ₙAr oder Cycloalkyl mit 3 bis 7 C-Atomen,
- Het: einen unsubstituierten oder einfach oder mehrfach durch A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A und/oder OOCR⁶ substituierten, gesättigten, ungesättigten oder aromatischen mono- oder bicyclischen heterocyclischen Rest, ausgewählt unter 1-Piperidyl, 1-Piperazyl, 1-(4-Methyl)-piperazyl, 4-Methylpiperazin-1-ylamin, 4-Morpholinyl, 1-Pyrrolidinyl, 1-Pyrazolidinyl 1-(2-Methyl)-Pyrazolidinyl, 1-Imidazolidinyl oder 1-(3-Methyl)-imidazolidinyl, Thiophen-2-yl oder Thiophen-3-yl, 2-, 3- oder 4-Pyridyl, das unsubstituiert oder durch eine oder mehrere CN-Gruppe substituiert sein kann, 2-, 4- oder 5-Oxaolyl, 2-, 4- oder 5-Thiazolyl, Chinolinyl, Isochinolinyl, 2- oder 4-Pyridazyl, 2-, 4 oder 5-Pyrimidyl, 2-oder 3- Pyrazinyl,
- Ar: einen unsubstituierten oder einfach oder mehrfach durch A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A und/oder OOCR⁶ substituierten aromatischen Kohlenwasserstofferest mit 6 bis 14 Kohlenstoffatomen,
- n: unabhängig voneinander 0, 1, 2, 3, 4 oder 5
- p, q: unabhängig voneinander 0, 1, 2 oder 3
- r: 0, 1, 2, 3, 4 oder 5,
- w: 0, 1, 2 oder 3
und
- Hal: F, Cl, Br oder I
bedeuten, und
- R⁸: für A, Hal, CN, NO₂, NH₂, CF₃, OCF₃ oder SO₂CH₃ steht,
sowie deren pharmazeutisch verwendbaren Derivate, Solvate und Stereoisomere, einschließlich deren Mischungen in allen Verhältnissen, und insbesondere deren Salze und Solvate.

Weiterhin bevorzugte Verbindungen der Formel I sind Verbindungen der Formel Id, 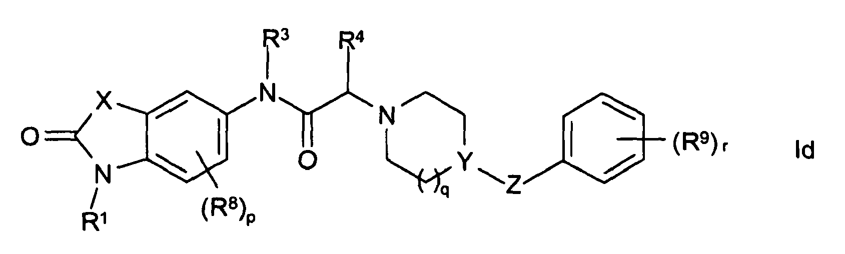 worin
- R¹, X, R³, R⁴, Y, Z, R⁸, p und q: die vor und nachstehend angegebenen Bedeutungen aufweisen,
und worin
- r: 0, 1, 2, 3, 4 oder 5, und die Reste
- R⁹: wie vorstehend definiert sind,
sowie deren pharmazeutisch verwendbaren Derivate, Solvate und Stereoisomere, einschließlich deren Mischungen in allen Verhältnissen, und insbesondere deren Salze und Solvate.

Gegenstand der vorliegenden Erfindung sind bevorzugt Verbindungen der Formel I wie vorstehend beschrieben, worin R¹, R², r, Ar und Het die vorstehend genannten Bedeutungen aufweisen, und worin
- A: geradkettiges Alkyl mit 1 bis 4 C-Atomen oder verzweigtes Alkyl mit 3 bis 6 C-Atomen,
- X: O oder N-R²,
- R³: H oder A,
- R⁴: H, A oder (CH₂)ₙAr,
- Y: CH, COR⁷ oder N,
- R⁷: H,
- Z: O, CH₂ oder CH₂CH₂,
- n: unabhängig voneinander 0, 1, 2 oder 3, und
- p, q: unabhängig voneinander 0, 1 oder 2
bedeuten, und
- R⁸, R⁹: unabhängig voneinander für F, Cl, Br, I, CF₃ oder OCF₃ stehen,
sowie deren Solvate und Salze.

Vorzugsweise ist die Summe aus n und m größer als Null.

Einige bevorzugte Gruppen von Verbindungen können durch die folgenden Teilformeln Iη) bis Iµ) ausgedrückt werden, die der Formel I, vorzugsweise der Formel Ic und besonders bevorzugt der Formel Id entsprechen und worin die nicht näher bezeichneten Reste die vorstehend angegebene Bedeutung haben, worin jedoch

| | | |
|---|---|---|
| in Iη) | R¹ | H bedeutet; |
| | | |
| in Iθ) | R¹ | H und |
| | X | O, S oder NR² bedeutet; |
| | | |
| in Il) | R¹ | H, |
| | X | O, S oder NR², |
| | R² | H, |
| | R³ | H oder A, und |
| | R⁹ | Hal bedeutet; |
| | | |
| in Iκ) | R¹ | H, |
| | X | O, S oder NR², |
| | R² | H, |
| | R³ | H oder A, |
| | Y | CH, COR⁶ oder N, und |
| | R⁹ | Hal bedeutet; |
| | | |
| in Iλ) | R¹ | H, |
| | X | O, S oder NR², |
| | R² | H, |
| | R³ | H oder A, |
| | Y | CH, COR⁶ oder N, |
| | R⁶ | H, |
| | Z | O, CH₂, CH₂CH₂ oder N-R¹⁵, |
| | R¹⁵ | H oder A, und |
| | R⁹ | Hal bedeutet; |
| | | |
| in Iµ) | R¹ | H, |
| | X | O, S oder NR², |
| | R² | H, |
| | R³ | H oder A, |
| | R⁴ | H oder A, |
| | Y | CH, COR⁶ oder N, |
| | R⁶ | H, |
| | Z | O, CH₂, CH₂CH₂ oder N-R¹⁵, |
| | R¹⁵ | H oder A, und |
| | R⁹ | Hal bedeutet. |

In einer bevorzugten Ausführungsform der vorliegenden Erfindung steht in den Teilformeln Iη) bis Iµ) der Rest R⁸ für A, Hal, insbesondere F oder Br, CN, NO₂ NH₂, CF₃, OCF₃ oder SO₂CH₃, besonders bevorzugt für A oder Hal; p für 0, 1 oder 2, besonders bevorzugt 0 oder 1; q für 0, 1 oder 2, besonders bevorzugt für 1 oder 2; und r für 0, 1, 2 oder 3, besonders bevorzugt für 0, 1 oder 2. Im Rahmen dieser bevorzugten Ausführungsform steht in den Teilformeln Iη) bis Iλ) der Rest R⁹ besonders bevorzugt für A, F, Cl, Br, I, CN, NO₂, NH₂, CF₃, OCF₃ oder SO₂CH₃ und insbesondere für F. Im Rahmen dieser bevorzugten Ausführungsform steht in den Teilformeln Iη) bis Iµ) der Rest R⁴ vorzugsweise für H, Methyl, Ethyl, Trifluormethyl, Pentafluorethyl, Propyl, Isopropyl, Butyl, Isobutyl, sek.-Butyl, tert.-Butyl oder Phenyl, besonders bevorzugt für H, Methyl, Ethyl, Trifluormethyl, Phenyl und insbesondere für H, Methyl oder Phenyl.

In einer speziellen und bevorzugten Ausführungsform betrifft die vorliegende Erfindung eine Verbindung der Formel I, worin X für O steht, sowie deren Solvate und Salze.

In einer weiteren speziellen und bevorzugten Ausführungsform betrifft die vorliegende Erfindung eine Verbindung der Teilformeln Iη) bis Iµ) wie vorstehend definiert, worin X für O steht, sowie deren Solvate und Salze.

In einer weiteren speziellen und bevorzugten Ausführungsform betrifft die vorliegende Erfindung eine Verbindung der Formel I, der Formel Ic, der Formel Id und der Teilformeln Iη) bis Iµ) wie vorstehend definiert, worin R⁴ ausgewählt ist unter H, substituierten oder unsubstituierten Alkylresten, vorzugsweise unsubstituierten Alkylresten, insbesondere Alkylresten mit eins bis vier Kohlenstoffatomen, und substituierten oder unsubstituierten Arylresten, vorzugsweise unsubstituierten Arylresten, unsubstituierten Phenylresten, sowie deren Solvate und Salze. Besonders bevorzugt ist R⁴ ausgewählt unter H, Methyl und Phenyl.

In einer weiteren speziellen und besonders bevorzugten Ausführungsform betrifft die vorliegende Erfindung eine Verbindung der Formel I, der Formel Ic, der Formel Id und inbesondere eine der Teilformeln Iη) bis Iµ) wie vorstehend definiert sowie deren Solvate und Salze, worin X für O steht und worin der Rest R⁸ für A, Hal, insbesondere F oder Br, CN, NO₂ NH₂, CF₃, OCF₃ oder SO₂CH₃, besonders bevorzugt für A oder Hal; p für 0, 1 oder 2, besonders bevorzugt 0 oder 1; q für 0, 1 oder 2, besonders bevorzugt für 1 oder 2; und r für 0, 1, 2 oder 3, besonders bevorzugt für 0, 1 oder 2, steht. Im Rahmen dieser speziellen und besonders bevorzugten Ausführungsform steht in den Teilformeln Iη) bis Iλ) der Rest R⁹ besonders bevorzugt für A, F, Cl, Br, I, CN, NO₂, NH₂, CF₃, OCF₃ oder SO₂CH₃ und insbesondere für F. Ferner steht im Rahmen dieser weiteren speziellen und besonders bevorzugten Ausführungsform in den Teilformeln Iη) bis Iλ) der Rest R⁴ vorzugsweise für H, Methyl, Ethyl, Trifluormethyl, Pentafluorethyl, Propyl, Isopropyl, Butyl, Isobutyl, sek.-Butyl oder tert.-Butyl, besonders bevorzugt für H, Methyl, Ethyl, Trifluormethyl, Pentafluorethyl oder Phenyl und insbesondere für H, Methyl oder Phenyl. Bevorzugt steht im Rahmen dieser weiteren speziellen und besonders bevorzugten Ausführungsform in den Teilformeln Iη) bis Iλ) der Rest R⁴ vorzugsweise für H, Methyl, Ethyl, Trifluormethyl, Pentafluorethyl, Propyl, Isopropyl, Butyl, Isobutyl, sek.-Butyl oder tert.-Butyl, besonders bevorzugt für H, Methyl, Ethyl, Trifluormethyl, Pentafluorethyl und insbesondere für H oder Methyl.

In einer weiteren ganz besonders bevorzugten Ausführungsform der vorliegenden Erfindung sind die Verbindungen der Formel I ausgewählt unter
g) 2-[4-(4-Fluoro-benzyl)-4-hydroxy-piperidin-1-yl]-N-(2-oxo-2,3-dihydro-benzooxazol-6-yl)-acetamide;
h) 2-[4-(4-Fluoro-phenoxy)-piperidin-1-yl]-N-(2-oxo-2,3-dihydrobenzooxazol-6-yl)-propionamide;
i) 2-[4-(4-Fluorobenzyl)-piperidin-1-yl]-N- (2-oxo-2,3-dihydrobenzooxazol-6-yl)-propionamide;
j) 2-(4-Benzyl-piperazin-1-yl)-N-(2-oxo-2,3-dihydrobenzooxazol-6-yl)-propionamide;
k) 2-[4-(4-Fluoro-benzyl)-4-hydroxy-piperidin-1-yl]-N-(2-oxo-2,3-dihydro-benzoxazol-6-yl)-propionamide;
l) 2-[4-(3,5-Difluoro-benzyl)-4-hydroxy-piperidin-1-yl]-N-(2-oxo-2,3-dihydro-benzoxazol-6-yl)-propionamide
m) 6-(2-(4-(4-Fluorbenzyl)-1-piperidyl)-acetamido)-2,3-dihydrobenzoxazol-2-on
n) 2-[4-(4-Fluoro-benzyl)-piperidin-1-yl]-N-(2-oxo-2,3-dihydrobenzooxazol-6-yl)-2-phenyl-acetamide
o) 2-[4-(4-Fluoro-phenoxy)-piperidin-1-yl]-N-(2-oxo-2,3-dihydrobenzooxazol-6-yl)-2-phenyl-acetamide
p) 2-[4-(2,4-Difluoro-benzyl)-piperidin-1-yl]-N-(2-oxo-2,3-dihydrobenzooxazol-6-yl)-propionamide
q) 2-(4-Benzyl-1-piperidyl)-N-(2-oxo-2,3-dihydrobenzoxazol-6-yl)-acetamid
r) N-(2-Oxo-2,3-dihydrobenzoxazol-6-yl)-2-(4-thiophen-2-ylmethyl-1-piperidyl)-acetamid
sowie deren pharmazeutisch verwendbaren Derivaten, Solvaten und Stereoisomeren, einschließlich deren Mischungen in allen Verhältnissen, und insbesondere deren Salzen und Solvaten.

Weiterhin bevorzugte Verbindungen der Formel I sind Verbindungen der Formel Ie, 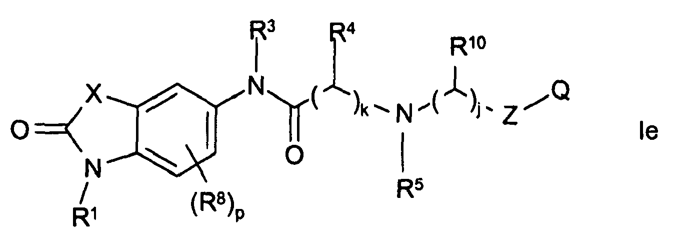 worin
- X, R¹, R³, R⁴, k, Z und Q: wie vorstehend und nachstehend definiert sind und worin
- R⁵: H oder A,
- R¹⁰: unabhängig H, A oder (CH₂)ₙAr,
und
- j: 2 oder 3 bedeutet,
sowie deren pharmazeutisch verwendbaren Derivate, Solvate und Stereoisomere, einschließlich deren Mischungen in allen Verhältnissen, und insbesondere deren Salze und Solvate.

Bevorzugte Verbindungen der Formel Ie sind Verbindungen der Formel If, 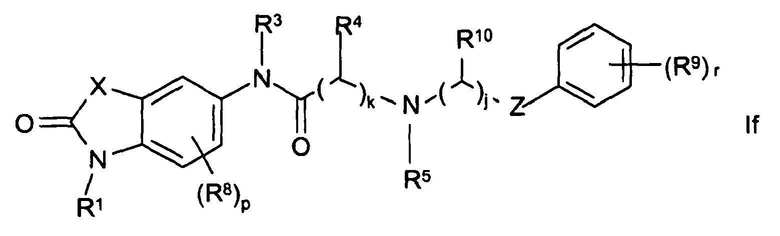 worin
- X, R¹, R³, R⁴, k, Z, R⁵, R¹⁰ und j: wie vorstehend und nachstehend definiert sind und worin
- r: 0, 1, 2, 3, 4 oder 5, bevorzugt 0, 1, 2 oder 3 und insbesondere 0, 1, 2 oder 3 bedeutet, und
- R⁹: unabhängig voneinander für Hal steht,
sowie deren pharmazeutisch verwendbaren Derivate, Solvate und Stereoisomere, einschließlich deren Mischungen in allen Verhältnissen, und insbesondere deren Salze und Solvate.

Gegenstand der vorliegenden Erfindung sind bevorzugt Verbindungen der Formel I und insbesondere Verbindungen der Formel If wie vorstehend beschrieben, worin
- A: geradkettiges Alkyl mit 1 bis 4 C-Atomen, verzweigtes Alkyl mit 3 bis 6 C-Atomen, Alkoxy mit 1 bis 4 Kohlenstoffatomen,
- X: O oder N-R², und
- R³: H oder A bedeutet,
- R⁴: unabhängig voneinander ausgewählt sind unter H, A oder (CH₂)ₙAr und
- R⁵: H oder A, bevorzugt H oder Alkyl, insbesondere H oder Methyl,
- Z: O, CH₂ oder CH₂CH₂, insbesondere CH₂,
- n: unabhängig voneinander 0, 1, 2 oder 3,
- p: 0, 1 oder 2, vorzugsweise 0 oder 1, besonders bevorzugt 0,
- k: 1 oder 2, vorzugsweise 1,
- j: 2 oder 3, vorzugsweise 3, und
- r: 0, 1 oder 2, vorzugsweise 1,
bedeuten, und
- R⁹: für F, Cl, Br, I, CF₃, OCF₃ und insbesondere für F steht
sowie deren Solvate und Salze.

Vorzugsweise ist die Summe aus n und m größer als Null.

Bevorzugte Verbindungen der Formel Ie bzw. If sind Verbindungen der Formel Ig, 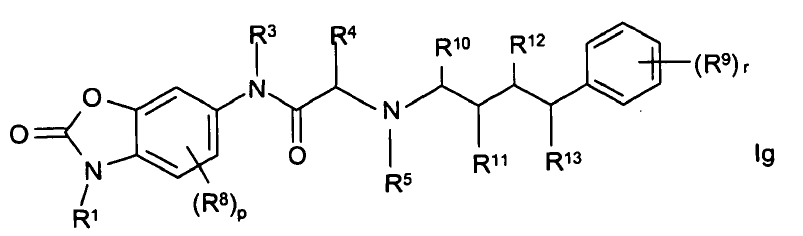 worin
- R¹, R³: H oder Methyl, vorzugsweise H,
- R⁴: H oder oder A, vorzugsweise H,
- R⁵: H oder oder A, vorzugsweise H oder Methyl,
und R¹⁰, R¹¹, R¹² und R¹³ unabhängig voneinander Ar, H oder A, vorzugsweise H oder A bedeuten, und insbesondere für H stehen.

Besonders bevorzugte Verbindungen der Formeln Ie bis Ig sind ausgewählt unter
s) 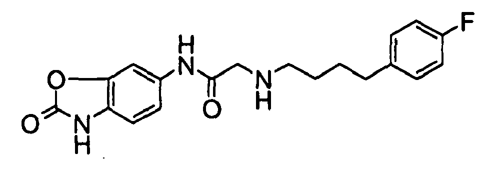 N-(2,3-Dihydro-2-oxobenzoxazol-6-yl)-2-[4-(4-fluorphenyl)-butylamino]-acetamid
t) 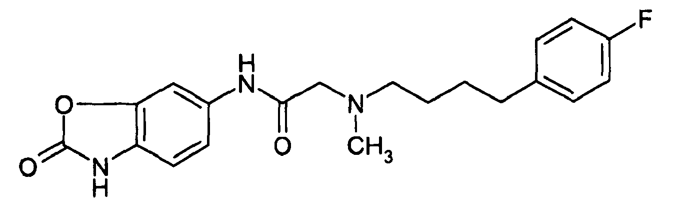 N-(2,3-Dihydrobenzoxazol-6-yl)-2-{N-[4-(4-fluorphenyl)-butyl]-N-methylamino}-acetamid,
   sowie deren pharmazeutisch verwendbaren Derivate, Solvate und Stereoisomere, einschließlich deren Mischungen in allen Verhältnissen, und insbesondere deren Salze und Solvate.

Die erfindungsgemäßen Verbindungen können, abhängig von der Auswahl der vorstehend beschriebenen Substituenten und Reste, ein oder mehrere chirale Zentren, insbesondere ein oder mehrere chirale Kohlenstoffatome, aufweisen. Wenn eine erfindungsgemäße Verbindung definierter Zusammensetzung ein oder mehrere chirale Zentren aufweist, kann diese Verbindung definierter Zusammensetzung in unterschiedlichen Stereoisomeren vorliegen. Gegenstand der vorliegenden Erfindung sind alle möglichen solchen Stereoisomere erfindungsgemäßer Verbindungen, die sowohl als einzelne, stereochemisch einheitliche Verbindungen, als auch als Gemische zweier oder mehrerer stereochemisch einheitlicher Verbindungen vorliegen können. Im Falle von Gemischen zweier oder mehrerer Stereoisomere können die einzelnen Stereoisomere in unterschiedlichen oder gleichen Anteilen vorliegen. Bei Gemischen aus zwei Stereoisomeren, ihn gleichen Anteilen vorliegen und optische Antipoden darstellen, spricht man von racemischen Gemischen. Racemische Gemische von Verbindungen der Formel I sind ebenfalls Gegenstand der vorliegenden Erfindung.

Gegenstand der vorliegenden Erfindung sind insbesondere Stereoisomere, die dadurch entstehen, dass der Rest R⁴ bzw. ein Rest R⁴ oder beide Reste R⁴ eine von H verschiedene Bedeutung aufweist.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart;) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe, z. B. die Verbindungen der Formel II, V und/oder VI, können, falls erwünscht, auch in situ gebildet werden, so daß man sie aus dem Reaktionsgemisch nicht isoliert, sondern sofort weiter zu der Verbindung der Formel I umsetzt.

Die Verbindungen der Formel I können vorzugsweise erhalten werden, indem man Verbindungen der Formel II mit Verbindungen der Formel V und der Formel VI umsetzt. Dabei ist die Reihenfolge der Umsetzung der Verbindungen grundsätzlich beliebig. Beispielsweise können die Verbindungen der Formel II, V und VI in einer Eintopfreaktion zu Verbindungen der Formel I umgesetzt werden. In Abhängigkeit von der Auswahl der Gruppen L¹, L², L³ und L⁴ kann jedoch eine bestimmte Reihenfolge bei der Umsetzung der Ausgangsverbindungen vorteilhaft sein. In vielen Fällen ist es vorteilhaft, die Ausgangsverbindung der Formel II zuerst mit einer Ausgangsverbindung der Formel V und das erhaltene Reaktionsprodukt anschließend mit einer Ausgangsverbindung der Formel VI umzusetzen, um zu einer Verbindung der Formel I zu gelangen (die Umsetzung in dieser Reihenfolge wird im folgenden als Variante 1 bezeichnet). Alternativ kann zuerst eine Ausgangsverbindung der Formel V mit einer Ausgangsverbindung der Formel VI umgesetzt werden und das erhaltende Reaktionsprodukt dieser Umsetzung mit einer Ausgangsverbindung der Formel II umgesetzt werden, um zu einer Verbindung der Formel I zu gelangen (die Umsetzung in dieser Reihenfolge wird im folgenden als Variante 2 bezeichnet).

In den Verbindungen der Formel II bedeutet L¹ vorzugsweise H oder eine die Aminofunktion aktivierende Gruppe, beispielsweise ein Metallion. Geeignete Metallionen sind insbesondere Alkalimetall-, Erdalkalimetall- oder Aluminium-lonen. Bevorzugt als Metallionen sind Alkalimetallionen, insbesondere Li, Na oder K. Bei mehrwertigen Metallionen bildet sich oft ein Komplex aus Metallion und zwei oder mehreren Verbindungen der Formel II, wobei der Komplex stöchiometrisch in der Regel so viele Verbindungen der Formel II umfasst, wie es der Wertigkeit des Metallion entspricht.

In den Verbindungen der Formel V bedeutet L² vorzugsweise Cl, Br, I, OH oder eine reaktionsfähig abgewandelte OH-Gruppe wie Alkylsulfonyloxy mit 1-6 C-Atomen (bevorzugt Methylsulfonyloxy oder Trifluormethylsulfoxy bzw. Trifluormethansulfonyloxy) oder Arylsulfonyloxy mit 6-10 C-Atomen (bevorzugt Phenyl- oder p-Tolylsulfonyloxy).

In den Verbindungen der Formel V bedeutet L³ vorzugsweise Cl, Br, I, OH oder eine dieses Kohlenstoffatom aktivierende Abgangsgruppe, beispielsweise eine Diazoniumgruppe.

In den Verbindungen der Formel VI bedeutet L⁴ vorzugsweise H oder eine die Aminofunktion aktivierende Gruppe, beispielsweise ein Metallion. Geeignete Metallionen sind insbesondere Alkalimetall-, Erdalkalimetall- oder Aluminium-lonen. Bevorzugt als Metallionen sind Alkalimetallionen, insbesondere Li, Na oder K. Bei mehrwertigen Metallionen zeigen die Verbindungen der Formel VI in der Regel ein ähnliches Verhalten wie vorstehend bei den Verbindungen der Formel II beschrieben.

Die Umsetzung gemäß Variante 1 ist insbesondere dann vorteilhaft, wenn in der Ausgangsverbindung V die Gruppen L² und/oder L³, bevorzugt L² und L³, für Halogen, insbesondere Cl, stehen und vorzugsweise in der Ausgangsverbindung II die Gruppe L¹ für H und/oder in der Ausgangsverbindung VI die Gruppe L⁴ für H steht. Besonders bevorzugt stehen in der Variante 1 die Gruppen L² und L³ für Halogen, insbesondere Cl, und die Gruppen L¹ und L⁴ für H.

Die Ausgangsverbindungen der Formeln II und VI sind in der Regel neu. Sie können aber nach an sich bekannten Methoden hergestellt werden. Die Ausgangsverbindungen der Formel V sind entweder neu oder literaturbekannt bzw. handelsüblich. In jedem Fall können sie jedoch nach an sich bekannten Methoden hergestellt werden.

Die Umsetzung der Verbindungen der Formel II mit Verbindungen der Formel V erfolgt in der Regel in einem inerten Lösungsmittel, vorzugsweise in Gegenwart eines säurebindenden Mittels. Als säurebindende Mittel kommen alle in der organischen Synthesechemie üblichen Basen, sowohl anorganische als auch organische, bevorzugt organische Basen, in Betracht. Beispiele für geeignete organische Basen sind Triethylamin, Diisopropylamin, Diisopropylethylamin (DIPEA), Dimethylanilin, Pyridin oder Chinolin. Auch der Zusatz einer anorganischen Base, wie beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums, kann günstig sein.

Die Reaktionszeit liegt je nach den angewendeten Bedingungen zwischen einigen Minuten und 14 Tagen, die Reaktionstemperatur zwischen etwa -30° und 180°, normalerweise zwischen -20°und 140°, bevorzugt zwischen -10° und 130° und insbesondere zwischen etwa 0° und etwa 120°. In vielen Fällen ist es günstig, die Umsetzung einer Ausgangsverbindung der Formel II mit einer Ausgangsverbindung der Formel V bei vergleichsweise niedrigen Temperaturen durchzuführen, beispielsweise bei einer Temperatur im Bereich von -20° bis 100°, bevorzugt 0° bis 80°und insbesondere 0° bis 50°, z. B. etwa bei Raumtemperatur (20°). Bei einer Umsetzung in diesem Temperaturbereich ist es in vielen Fällen günstig, eine organische Base wie Triethylamin oder Diisopropylamin zu verwenden. In vielen Fällen ist es günstig, das bei der Umsetzung von Verbindungen der Formel II mit Verbindungen der Formel V erhaltende Reaktionsprodukt bei bei vergleichsweise niedrigen Temperaturen durchzuführen, beispielsweise bei einer Temperatur im Bereich von -20° bis 100°, bevorzugt 0° bis 80°und insbesondere 0° bis 50°, z. B. etwa bei Raumtemperatur (20°). Bei einer Umsetzung in diesem Temperaturbereich ist es in vielen Fällen günstig, eine organische Base wie Triethylamin, Diisopropylethylamin oder Diisopropylamin zu verwenden. In vielen Fällen ist es günstig, das bei der Umsetzung von Verbindungen der Formel II mit Verbindungen der Formel V erhaltende Reaktionsprodukt bei höheren Temperaturen mit einer Ausgangsverbindung der Formel VI umzusetzen. Geeignete höhere Temperaturen liegen beispielsweise im Bereich von 20° bis 170°, bevorzugt 60° bis 150°und insbesondere 80° bis 130°, z. B. bei etwa 120°. Bei einer Umsetzung in diesem Temperaturbereich ist es in vielen Fällen günstig, eine organische Base wie Pyridin oder Chinolin einzusetzen, die erst bei höheren Temperaturen siedet.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, Wasser oder Gemische der genannten Lösungsmittel.

In vorteilhafter Weise kann eine Umsetzung einer Verbindung der Formel II mit einer Verbindung der Formel V bei vergleichsweise niedrigen Temperaturen in einem niedrig siedenten inerten Lösungsmittel, z. B. Kohlenwasserstoffen, wie Petrolether oder Hexan, oder chlorierten Kohlenwasserstoffen, wie Chloroform oder Dichlormethan, durchgeführt werden.

In vorteilhafter Weise kann eine Umsetzung des Reaktionsprodukts aus einer Verbindung der Formel II und einer Verbindung der Formel V mit einer Verbindung der Formel VI bei vergleichsweise niedrigen Temperaturen in einem polaren inerten Lösungsmittel, z. B. chlorierten Kohlenwasserstoffen, wie Dichlormethan und Chloroform, Dimethylformamid (DMF), Acetonitril, Sulfoxiden, wie Dimethylsulfoxid (DMSO), und insbesondere in Acetonitril durchgeführt werden.

Eine Umsetzung des Reaktionsprodukts aus einer Verbindung der Formel II und einer Verbindung der Formel V mit einer Verbindung der Formel VI bei höheren Temperaturen kann vorteilhaft in einem höher siedenten inerten Lösungsmittel, z. B. Kohlenwasserstoffen, wie Benzol, Toluol oder Xylol, chlorierten Kohlenwasserstoffen, wie Trichlorethylen oder 1,2-Dichlorethan, Glykolethern, wie Ethylenglykolmonomethyl- oder - monoethylether (Methylglykol oder Ethylglykol) oder Ethylenglykoldimethylether (Diglyme), sowie Dimethylformamid (DMF), Acetonitril, Sulfoxiden, wie Dimethylsulfoxid (DMSO), oder Nitroverbindungen, wie Nitromethan oder Nitrobenzol, durchgeführt werden. Die Umsetzung bei höheren Temperaturen kann vorteilhaft in einer höher siedenten organischen Base, wie Pyridin oder Chinolin, an Stelle eines Lösungsmittels durchgeführt werden.

In vielen Fällen kann die Umsetzung der Ausgangsverbindungen der Formel II, der Formel V und/oder der Formel VI beziehungsweise die Umsetzung des Reaktionsprodukts aus einer Verbindung der Formel II und einer Verbindung der Formel V mit einer Verbindung der Formel VI oder die Umsetzung des Reaktionsprodukts aus einer Verbindung der Formel V mit einer Verbindung der Formel VI mit einer Verbindung der Formel II vorteilhaft in wenigstens einer organischen Base, vorzugsweise wenigstens einer der vorstehend genannten organischen Basen, an Stelle eines Lösungsmittels durchgeführt werden.

Gegenstand der Erfindung sind auch die optisch aktiven Formen (Stereoisomeren), die Enantiomeren, die Racemate, die Diastereomeren sowie die Hydrate und Solvate der erfindungsgemäßen Verbindungen. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen oder Prodrug-Derivate.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden. Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen der Formel I, z.B. Gemische zweier Diastereomere z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trichloressigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Auch physiologisch unbedenkliche organische Basen, wie z.B. Ethanolamin, können verwendet werden.

Die Erfindungsgemäßen Verbindungen können als Therapeutika, Diagnostika und/oder Kosmetika beziehungsweise zusammen mit einem oder mehreren von den erfindungsgemäßen Verbindungen verschiedenen Wirkstoffen und/oder Hilfsstoffen in Therapeutika, Diagnostika und oder Kosmetika verwendet werden. Üblicherweise werden die erfindungsgemäßen Verbindungen in Form von pharmazeutischen, diagnostischen und/oder kosmetischen Formulierungen eingesetzt. Solche Formulierungen und Verfahren zu ihrer Herstellung sind dem Fachmann bekannt.

Beispiele für solche Formulierungen sind wenigstens eine erfindungsgemäße Verbindung enthaltende Suspensionen, Emulsionen, Lösungen, Liposome, Salzen, Pasten, bioabbaubare Polymere, Nanopartikel, Tabletten, beschichtete Tabletten, Dragees, Filmtabletten, Kapseln, Pillen, Granulate, Pulver, Aerosole, Tropfen oder Sprays.

Die erfindungsgemäßen Verbindungen bzw. Formulierungen, die wenigstens eine erfindungsgemäße Verbindung enthalten, können an Mensch oder Tier verabreicht werden, z. B. lokal oder systemisch und insbesondere oral, intravenös, intraperitoneal, subkutan, transdermal, nasal, buccal und/oder ionotophoretisch.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend eine wirksame Menge mindestens einer der Verbindungen der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z. B. ein oder mehrere Vitamine.

Für die Applikation als Inhalationsspray können Sprays verwendet werden, die den Wirkstoff entweder gelöst oder suspendiert in einem Treibgas oder Treibgasgemisch (z. B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Gegenstand der vorliegenden Erfindung ist daher ein Verfahrem zur Herstellung pharmazeutischer Zubereitungen, das dadurch gekennzeichnet ist, daß man eine Verbindung der Formel I nach Anspruch 1 und/oder eines ihrer physiologischen unbedenklichen Salze und/oder eines ihrer Solvate zusammen mit mindestens einem festen, flüssigen oder halbflüssigen Träger- oder Hilfsstoff in eine geeignete Dosierungsform bringt.

Die Verbindungen der Formel I und/oder ihre physiologisch unbedenklichen Salze können als exzitatorische Aminosäure-Antagonist bei der Bekämpfung von Krankheiten, insbesondere zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen verwendet werden.

Gegenstand der vorliegenden Verbindung ist daher die Verwendung von Verbindungen der Formel I nach Anspruch 1 und/oder ihrer physiologisch unbedenklichen Salze oder Solvate zur Herstellung eines Arzneimittels zur Prophylaxe und/oder Behandlung von Schizophrenie, Depression, Demenz, Parkinsonschen Krankheit, Morbus Alzheimer, Lewy Bodies Dementia, Huntington, Tourette Syndrom, Angst, Lern- und Erinnerungseinschränkungen, neurodegenerativen Erkrankungen und anderen kognitiven Beeinträchtigungen, sowie Nikotinabhängigkeit und Schmerzen.

Dabei können die erfindungsgemäßen Verbindungen in der Regel in Analogie zu anderen bekannten Verbindungen mit ähnlichem Wirkprofil, wie z.B. Ifenprodil, verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die erfindungsgemäßen Verbindungen zeigen bei vergleichsweise leichter Herstellbarkeit ein vorteilhaftes Wirkprofil. So zeichnen sich die erfindungsgemäßen Verbindungen vorzugsweise als Polyamin-Antagonisten mit selektiver Bindung am NR2B-Rezeptor der NMDA-Unterrezeptorklasse mit einer vorzugsweise sehr geringen QT-Verlängerung zur Behandlung der wie vorstehend beschriebenen Krankheiten aus. Ferner zeigen erfindungsgemäße Verbindungen in Rezeptorbindungstests eine Affinität zur Ifenprodil-Bindungsstelle des NMDA-Rezeptors vorzugsweise bereits in nanomolaren Konzentrationen.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet gegebenenfalls die organische Phase, z. B. über Natriumsulfat, dampft die organische Phase ein und reinigt den erhaltenen Rückstand durch Chromatographie, z. B. an Kieselgel, und/oder durch Kristallisation. Wenn nicht anders angegeben, werden HPLC-Analysen auf einer 3µ Silica-Rod-Säule mit einem 210-sekündlichem Gradienten von 20% bis 100% mit Wasser/Acetonitril/0,01 % Trifluoressigsäure als Eluenten bei einem Fluss von 2,2 ml/min durchgeführt. Die Detektion erfolgt bei einer Wellenlänge von 220 nm.

### Beispiel 1

### Synthese von 3-[4-(Fluorbenzyl)-1-piperidyl]-N-(2-oxo-2,3-dihydrobenzoxazol-6yl)-propionamid

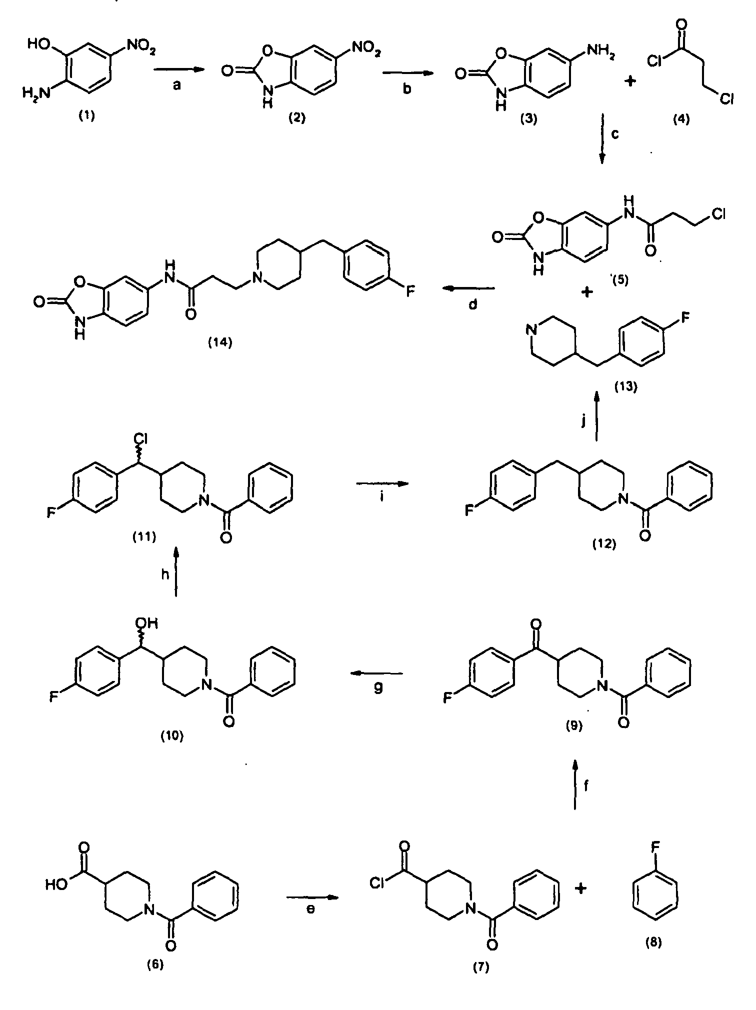

a) Eine Lösung von 30,83 g (0,2 mol) 2-Hydroxy-4-nitroanilin und 48,64 g (0,3 mol) Carbonyldiimidazol in 400 ml getrocknetem THF wird drei Stunden unter Erhitzen zum Rückfluss gerührt. Zur Aufarbeitung wird das Lösungsmittel abdestilliert, der Rückstand im Ultraschallbad 30 Minuten mit 300 ml 1 N Salzsäure behandelt, der Niederschlag im Vakuum abfiltriert, wie 30 Wasser und wenig Methanol gewaschen und im Vakuum bei 50 °C getrocknet. Ausbeute: 33,3 g (92%); Schmelzpunkt: 246-249 °C.
b) Eine Lösung von 225 g Nitrobenzoxazolon (2) in 2,3 Litern Methanol wird mit 22 g 5-prozentiger Palladium/Aktivkohle versetzt und über Nacht in einer Wasserstoffatmosphäre hydriert. Anschließend wird der Katalysator abfiltriert, der Filterkuchen mit einer Mischung aus Dichlormethan/Methanol (9:1) gewaschen und das Filtrat vom Lösungsmittel befreit. Der verbleibende Rückstand wird in heißen Methanol gelöst, eingekühlt und der entstandene kristalline Feststoff im Vakuum filtriert. Der kristalline Feststoff wird mit kalten Methanol und Diethylether gewaschen und in Vakuum bei 50 °C getrocknet. Ausbeute: 169 g (100%); Schmelzpunkt: 206-208 °C
c) Eine Suspension von 3,32 g (0,022 mol) Aminobenzoxazolidinon in 30 ml Dichlormethan wird unter Rühren mit 2,46 g (0,024 mol) Triethylamin versetzt. Anschließend wird das Reaktionsgemisch unter Rühren und Kühlen so mit 3-Chlorpropionylchlorid, gelöst in 10 ml Dichlormethan, versetzt, dass die Temperatur 20 °C nicht überschreitet. Nach beendeter Zugabe wird noch 24 Stunden unter Kühlen gerührt. Zur Aufarbeitung wird das Reaktionsgemisch im Vakuum filtriert, der erhaltenen kristalline Rückstand mit Wasser verrührt, nochmals im Vakuum filtriert und getrocknet. Ausbeute: 1,27 g (24%); Schmelzpunkt: 237 °C.
d) Eine Suspension von 1,19 g (4,9 mmol) des unter Schritt c) erhaltenen Alkylchlorids (5) in 20 ml Acetonitril wird mit 1,10 Gramm (10,9 mmol) Triethylamin und 1,25 Gramm (5,4 mmol) des gemäß j) erhaltenen 4-Fluor-4-benzylpiperidins (13) versetzt und 24 Stunden bei Raumtemperatur gerührt. Der entstehende Niederschlag wird abfiltriert, in Dichlormethan aufgenommen, mit Wasser gewaschen, getrocknet und am Rotationsverdampfer bis zur Trockene eingeengt. Der Rückstand wird mit wenig Diethylether verrieten, im Vakuum filtriert und im Vakuum bei 50 °C getrocknet. Ausbeute: 1,32 g (67 %); Schmelzpunkt: 192-194 °C.
e) In einem 11-Dreihalskolben, versehen mit Magnetrührer, kühler, Tropftrichter und Gasableitungsrohr werden 34,99 g (15 mmol) Benzoylpiperidin-4-Carbonsäure (**6**) in 100 ml Dichlormethan gelöst und unter Rühren mit 16,32 ml Thionylchlorid versetzt. Das Reaktionsgemisch wird anschließend zwei Stunden zum Rückfluß erhitzt, nach dem Abkühlen im Rotationsverdampfer vom Lösungsmittel befreit und über Nacht im Kühlschrank gelagert. Das so erhaltene Produkt kann ohne weitere Reinigung weiter umgesetzt werden.
f) Zu einer Suspension von 101 g Aluminiumtrichlorid in 160 ml Dichlormethan werden unter Feuchtigkeitsausschluss (N₂-Atmosphäre) und bei Raumtemperatur ohne erkennbare Wärmetönung 42,5 ml Fluorbenzol zugetropft. Anschließend tropft man eine Lösung von 78,0 g Benzoylpiperidin-4-carbonsäurechlorid (**7**) in 160 ml Dichlormethan so zu, dass die Gasentwicklung nicht zu heftig erfolgt und die Innentemperatur 35 °C nicht übersteigt (ca. 20 min.) und rührt weitere 1,5 h bei Raumtemperatur. Zur Aufarbeitung wird das Reaktionsgemisch auf Eis (ca. 1 kg) gegossen, zweimal mit 350 ml Dichlormethan extrahiert und die vereinigten organischen Phasen nacheinander mit 1 N HCl (400 ml), halb gesättigter Natriumhydrogencarbonat-Lösung (400 ml, ca. 4-prozentig) und Wasser (400 ml) gewaschen, mit Natriumsulfat getrocknet und im Vakuum filtriert. Der Filterrückstand wird mit Dichlormethan gewaschen und das Lösungsmittel Abdestillieren. Der erhaltene Rückstand (107 g) wurde mit 200 ml Methyl-t-butylether kristallisiert. Ausbeute: 270,6 g; Schmelzpunkt: 128-129 °C.
g) Eine Suspension von 93,3 g der gemäß f) erhaltenen Verbindung (9) in 375 ml Methanol wird unter Eis/Wasser-Kühlung in ca. 30 min. portionsweise mit 4,0 g NaBH₄ versetzt, dass die Temperatur 13 °C nicht überschreitet. Nach beendeter Zugabe wird das Reaktionsgemisch noch ca. 30 min. die unter Kühlung gerührt. Anschließend werden 375 ml Eis/Wasser zugegeben, 10 Minuten gerührt und und einmal mit 300 ml und zweimal 100 ml Dichlormethan extrahiert. Die vereinigten organischen Phasen werden mit 300 ml Wasser gewaschen und mit Natriumsulfat getrocknet. Anschließend wird das Lösungsmittel im Vakuum entfernt. Der erhaltene Rückstand wird aus Methyl-t-butylether umkristallisiert. Ausbeute:91,2 g; Schmelzpunkt 116-117,5 °C
h) Eine Lösung von 88,3 g des unter g) erhaltenen Alkohols (10) in 425 ml Dichlormethan wird unter Feuchtigkeitsausschluss bei einer Temperatur im Bereich von 16-18 °C innerhalb von ca. 10 Minuten mit 31 ml SOCl₂ versetzt und anschließend eine weitere Stunde bei Raumtemperatur gerührt. Anschließend wird das Lösungsmittel im Vakuum entfernt, der Rückstand in 175 ml Diethylether gelöst und mit Petrolether (Sdp. 40-60 °C; ca. 35 ml) bis zur Trübungsschwelle versetzt. Nachbeendeter Kristallisation wird das Produkt abgesaugt und im Vakuum bei 50 °C getrocknet. Ausbeute:89,7 g; Schmelzpunkt: 115,5-117 °C
i) 2,1 kg der gemäß h) erhaltenen Verbindung (11), gelöst in 25 I THF, werden mit 1,1 kg Palladium/Aktivkohle (5-%ig) versetzt und unter Rühren bei einem Druck von 5 bar in einer Wasserstoffatmosphäre bei Raumtemperatur über Nacht hydriert. Danach wird das Reaktionsgemisch im Vakuum filtriert, der Rückstand mit 25 I THF nachgewaschen und das Filtrat im Vakuum vom Lösungsmittel befreit. Das so erhaltene Rohprodukt kann direkt weiter umgesetzt werden.
j) 2,89 g der gemäß i) erhaltenen Verbindung (12) werden mit 10 ml konzentrierter Salzsäure und 5 ml Wasser unter Rühren zu Stunden zum Rückfluß erhitzt. Anschließend wird die Reaktionslösung mit 50 ml Wasser verdünnt und mit Methyl-t-butylether extrahiert (3 x 30 ml). Die wässrige Phase wird mit Natriumhydroxid alkalisch gestellt und mit Dichlormethan extrahiert (3 x 30 ml). Die vereinigten Dichlormethan-Extrakte werden über Natriumsulfat getrocknet und vom Lösungsmittel befreit. Der erhaltene Rückstand wird in wenig Aceton gelöst und mit Ether/HCl versetzt. Der gebildete kristalline Niederschlag wird abfiltriert und anschließend im Vakuum bei 50 °C getrocknet. Ausbeute: 1,98 g; Schmelzpunkt: 121-130 °C

### Beispiel 2

Analog zu den in Beispiel 1 beschriebenen Verfahren können folgende Verbindungen erhalten werden:
a) 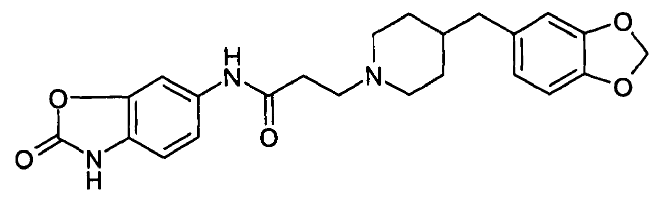 3-[4-(3,4-Methylendioxybenzyl)-piperidino]-N-(2-oxo-2,3-dihydrobenzoxazol-6-yl)-propionamid
b) 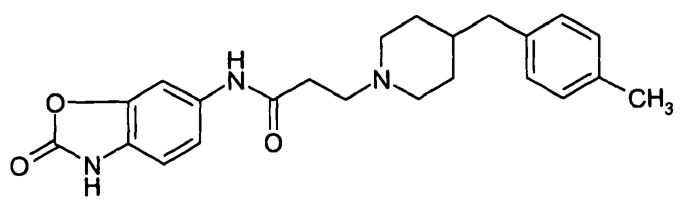 3-[4-(4-Methylbenzyl)-piperidino]-N-(2-oxo-2,3-dihydrobenzoxazol-6-yl)-propionamid
c) 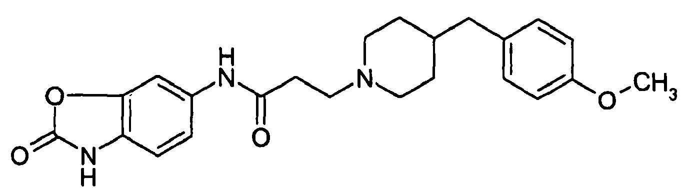 3-[4-(4-Methoxybenzyl)-1-piperidyl]-N-(2-oxo-2,3-dihydrobenzoxazol-6-yl)-propionamid
d) 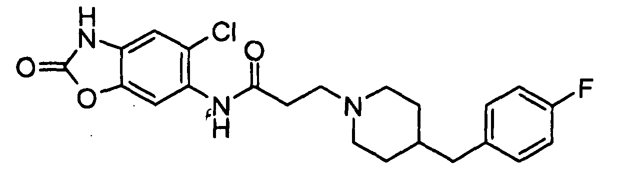 5-Chlor-6-[3-(4-(4-fluorbenzyl)-1-piperidyl)-propionamido]-2,3-dihydrobenzoxazol-2-on
e) 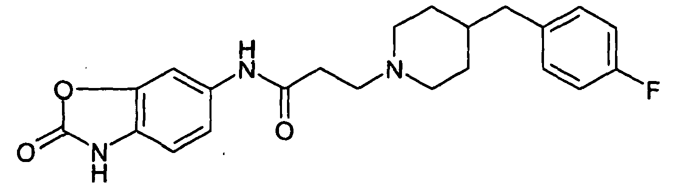 3-[4-(4-Fluorbenzyl)-1-piperidyl]-N-(2-oxo-2,3-dihydrobenzoxazol-6-yl)-propionamid

Physikalische Konstanten und analytische Daten (Massenspektrometrische Daten (FAB-MS) und Retensionszeiten (HPLC) der wie vorstehend synthetisierten Verbindungen sind in Tabelle I zusammengefasst.

**Tabelle I**

| **Struktur** | **Mol-masse** | **Smp.** |
|---|---|---|
| 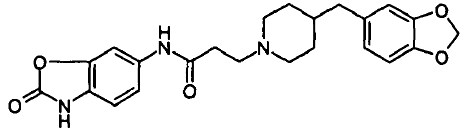 | 459,93* | 236-8* |
| 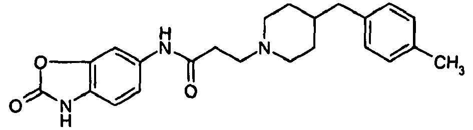 | 429,95* | 245-6* |
| 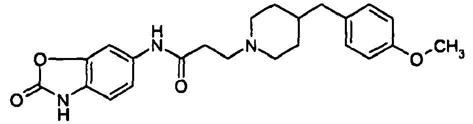 | 445,95* | 228-38* |
| 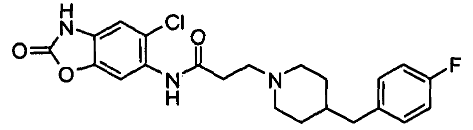 | 431,90 | 158-61 |
| 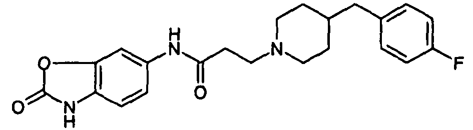 | 433,91* | 258-9* |

| | | |
|---|---|---|
| * Molmasse bzw. Schmelzpunkt des Hydrochlorids | | |

### Beispiel 3

Eine Suspension von 5-Aminobenzoxazolidinon in Dichlormethan wird unter Rühren mit 2-Chlorpropionsäurechlorid, gelöst Dichlormethan, versetzt und nach beendeter Zugabe noch 15 Minuten bei Raumtemperatur gerührt. Zur Aufarbeitung wird das Lösungsmittel im Vakuum entfernt und der erhaltene Rückstand durch chromatographische Methoden aufgearbeitet. Man erhält N-(2-oxo-2,3-dihydro-benzooxazol-6-yl)-2-chlor-propionamid.

Das erhaltene N-(2-oxo-2,3-dihydro-benzooxazol-6-yl)-2-chlor-propionamid und 4-(4-Fluorbenzyl)-piperidine werden in Pyridin gelöst und anschließend drei Stunden unter Rühren auf 120 °C erhitzt. Anschließend entfernt man das Lösungsmittel im Vakuum und arbeitet den Rückstand wie üblich auf, in dem man den Rückstand zwischen wenig Wasser und methylacetat verteilt. Die wässrige Phase wird mit Dichlormethan extrahiert, die vereinigten organischen Phasen über Natriumsulfat getrocknet und das Lösungsmittel im Vakuum entfernt. Der so erhaltene Rückstand wird durch Chromatographie gereinigt (mit Dichlormethan/Methanol im Verhältnis 97/3 als Eluenten). Man erhält 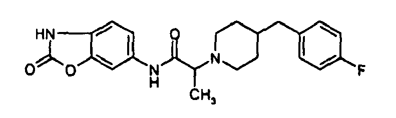 2-[4-(4-Fluorbenzyl)-piperid in-1 -yl]-N-(2-oxo-2,3-d ihydro-benzooxazol-6-yl)-propionamid.

### Beispiel 4

Analog zu den in Beispiel 3 beschriebenen Verfahren können folgende Verbindungen erhalten werden: 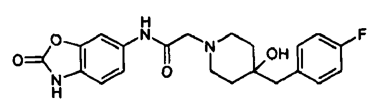 2-[4-(4-Fluor-benzyl)-4-hydroxy-piperidin-1-yl]-N-(2-oxo-2,3-dihydro-benzooxazol-6-yl)-acetamid; 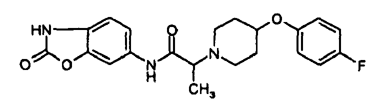 2-[4-(4-Fluor-phenoxy)-piperid in-1-yl]-N-(2-oxo-2,3-dihydrobenzooxazol-6-yl)-propionamid; 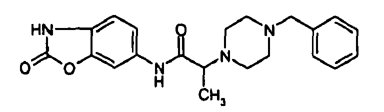 2-(4-Benzyl-piperazin-1-yl)-N-(2-oxo-2,3-dihydro-benzooxazol-6-yl)-propionamid; 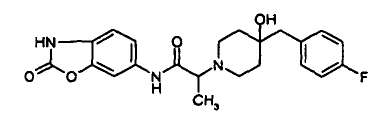 2-[4-(4-Fluor-benzyl)-4-hydroxy-piperidin-1-yl]-N-(2-oxo-2,3-dihydro-benzoxazol-6-yl)-propionamid; 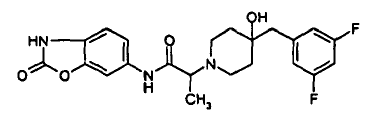 2-[4-(3,5-Difluor-benzyl)-4-hydroxy-piperidin-1-yl]-N-(2-oxo-2,3-dihydro-benzoxazol-6-yl)-propionamid. 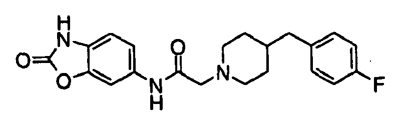 6-(2-(4-(4-Fluorbenzyl)-1-piperidyl)-acetamido)-2,3-dihydrobenzoxazol-2-on 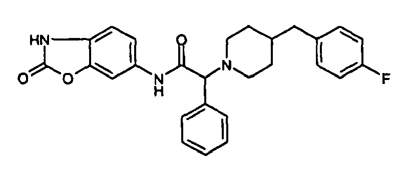 2-[4-(4-Fluoro-benzyl)-piperidin-1-yl]-N-(2-oxo-2,3-dihydrobenzooxazol-6-yl)-2-phenyl-acetamide 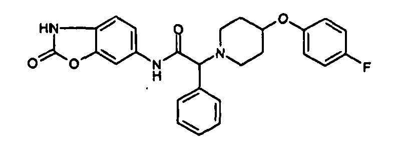 2-[4-(4-Fluoro-phenoxy)-piperidin-1-yl]-N-(2-oxo-2,3-dihydrobenzooxazol-6-yl)-2-phenyl-acetamide 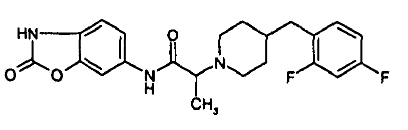 2-[4-(2,4-Difluoro-benzyl)-piperidin-1-yl]-N-(2-oxo-2,3-dihydrobenzooxazol-6-yl)-propionamide 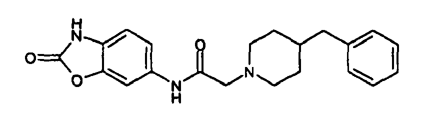 2-(4-Benzyl-1-piperidyl)-N-(2-oxo-2,3-dihydrobenzoxazol-6-yl)-acetamid 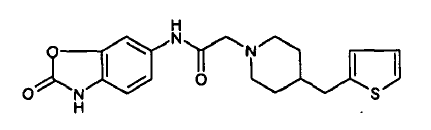 N-(2-Oxo-2,3-dihydrobenzoxazol-6-yl)-2-(4-thiophen-2-ylmethyl-1-piperidyl)-acetamid

Die Verbindungen können durch HPLC-Chromatographie gereinigt und/oder charakterisiert werden. Die Charakterisierung der Verbindungen über die Retensionszeit (Rₜ) kann auf einer Säule 3µ Silica-Rod mit einem 210 Sekunden Gradienten von 20 bis 100 % Wasser/Acetonitril/0,01% Trifluoressigsäure bei einem Fluss von 2,2 ml/Minute und einer Detektion bei einer Wellenlänge von 220 Nanometer erfolgen.

Physikalische Konstanten und analytische Daten (Massenspektrometrische Daten (FAB-MS) und Retensionszeiten (HPLC) der wie vorstehend synthetisierten Verbindungen sind in Tabelle II zusammengefasst.

**Tabelle II**

| | | | |
|---|---|---|---|
| **Struktur** | **MW g/mol** | **FAB-MS [M+H] gefunden** | **Rt(HPLC)** /**min** |
| 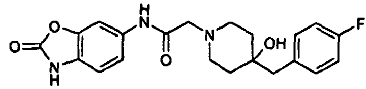 | 399.43 | 400.15 | 0.803 |
| 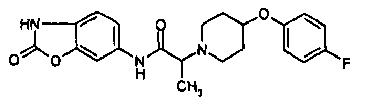 | 399.43 | 400.15 | 1.031 |
| 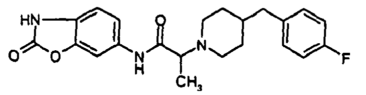 | 397.45 | 398.15 | 1.073 |
| 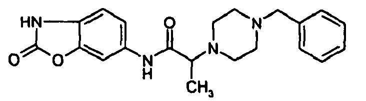 | 380.45 | 381.15 | 0.535 |
| 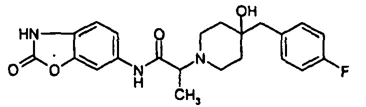 | 413.45 | 414.10 | 0.792 |
| 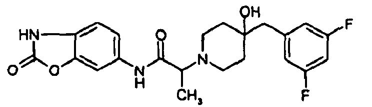 | 431.44 | 432.05 | 0.883 |
| 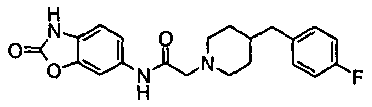 | 383,43 | 384,2 | |
| 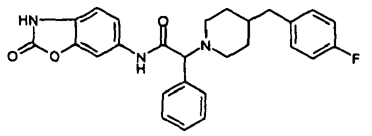 | 459, 52 | 460,2 | |
| 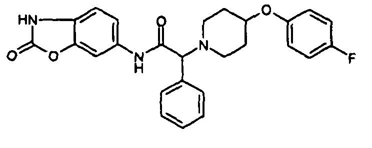 | 461,5 | 462,2 | |
| 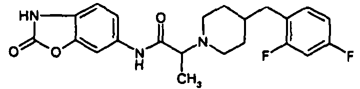 | 415,44 | 416,2 | |
| 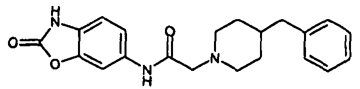 | 365,44 | 366,2 | |
| 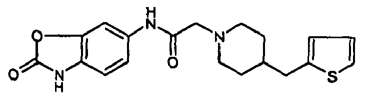 | 371,46 | 372,2 | |

### Beispiel 5

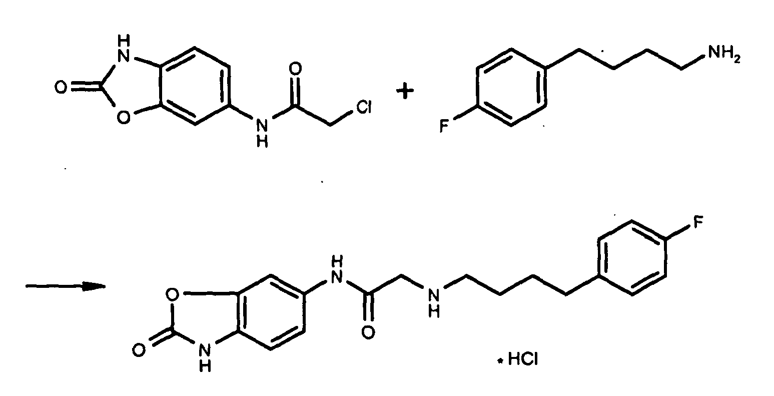

In einem 100 ml Rundkolben, versehen mit Magnetrührer, Kühler und Trockenröhrchen, wurden 1,13 g Chlorid (5) in 20 ml Acetonitril suspendiert, mit 1,12 g (4-F-Phenyl)butylamin und 1,73 ml Triethylamin versetzt und 2 h unter Rückfluß gerührt. Es wurde in Wasser gegossen, mit Dichlormethan extrahiert, getrocknet, filtriert und zum Rückstand abgezogen. Der Rückstand wurde über Kieselgel mit Dichlormethan + 4% Methanol chromatographiert und aus Methanol/Ether kristallisiert. Es wurde abgesaugt und getrocknet.

Die Base (0,41 g) wurde in Ethanol suspendiert, mit etherischer HCl versetzt, wobei kurzzeitig eine klare Lösung entstand, und das kristallisierte Salz abgesaugt und getrocknet.
Ausbeute: 230 mg (11,7 %)

### Beispiel 6

Analog zu dem in Beispiel 5 beschriebenen Verfahren können folgende Verbindungen erhalten werden: 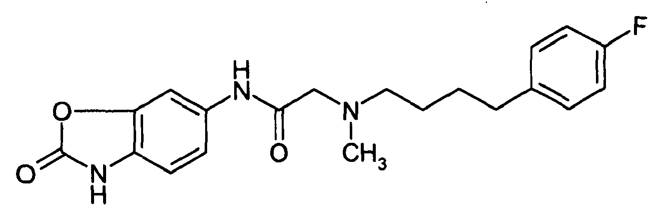 N-(2,3-Dihydrobenzoxazol-6-y1)-2-{N-[4-(4-fluorphenyl)-butyl]-N-methylamino}-acetamid.

Die nachfolgenden Beispiele betreffen pharmazeutische Zubereitungen:

### Beispiel A: Injektionsgläser

Eine Lösung von 100 g des Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g des Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g des Wirkstoffes der Formel I, 9,38 g NaH₂PO₄· 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg des Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 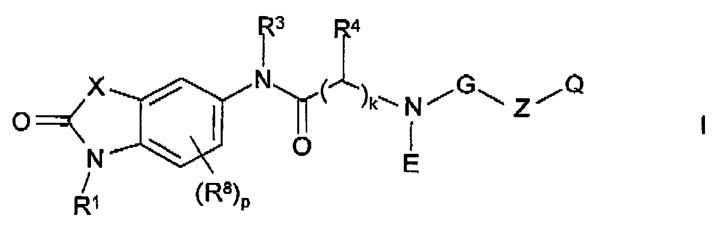 worin
R¹ H oder A
A geradkettiges oder verzweigtes Alkyl mit 1 bis 10 C-Atomen, Alkenyl mit 2 bis 10 C-Atomen, Alkoxy mit 1 bis 10 C-Atomen oder Alkoxyalkyl mit 2 bis 10 C-Atomen,
X O, S, N-R², CH₂ oder CH₂CH₂,
R² H oder A und
R³ H oder A bedeutet,
R⁴ unabhängig ausgewählt ist unter H, A oder (CH₂)ₙAr,
R⁶ für H, A oder Cycloalkyl mit 3 bis 7 C-Atomen, und
R⁸ A, Hal, CN, NO₂, NH₂, CF₃, OCF₃ oder SO₂CH₃, und
k für 1 oder 2 steht,
die Gruppe 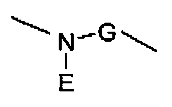 ausgewählt ist unter den Gruppen 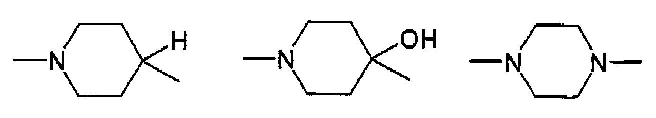 und 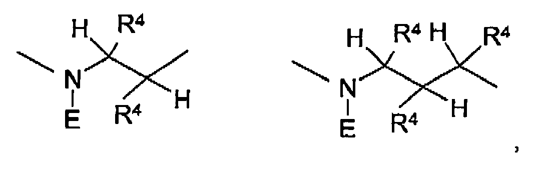
E H, A, (CH₂)ₙHet, (CH₂)ₙAr oder Cycloalkyl mit 3 bis 7 C-Atomen bedeutet,
Z O, S, N-R¹⁵, CH₂ oder CH₂CH₂,
R¹⁵ H, A, (CH₂)ₙHet. (CH₂)ₙAr oder Cycloalkyl mit 3 bis 7 C-Atomen,
Het einen unsubstituierten oder einfach oder mehrfach durch A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A und/oder OOCR⁶ substituierten, gesättigten, ungesättigten oder aromatischen mono- oder bicyclischen heterocyclischen Rest, ausgewählt unter 1-Piperidyl, 1-Piperazyl, 1-(4-Methyl)-piperazyl, 4-Methylpiperazin-1-ylamin, 4-Morpholinyl, 1-Pyrrolidinyl, 1-Pyrazolidinyl 1-(2-Methyl)-pyrazolidinyl, 1-Imidazolidinyl oder 1-(3-Methyl)-imidazolidinyl,Thiophen-2-yl oder Thiophen-3-yl, 2-, 3- oder 4-Pyridyl, das unsubstituiert oder durch eine oder mehrere CN-Gruppe substituiert sein kann, 2-, 4- oder 5-Oxazolyl, 2-, 4- oder 5-Thiazolyl, Chinolinyl, Isochinolinyl, 2- oder 4-Pyridazyl, 2-, 4- oder 5-Pyrimidyl, 2- oder 3-Pyrazinyl,
Ar einen unsubstituierten oder einfach oder mehrfach durch A, Hal, NO₂, CN, GR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A und/oder OOCR⁶ substituierten aromatischen Kohlenwasserstofferest mit 6 bis 14 Kohlenstoffatomen,
n 0, 1, 2, 3, 4 oder 5,
p 0, 1, 2 oder 3,
w 0, 1, 2 oder 3,
und
Hal F, Cl, Br oder I
bedeuten, und die Gruppe
Z-Q ausgewählt ist unter den Gruppen 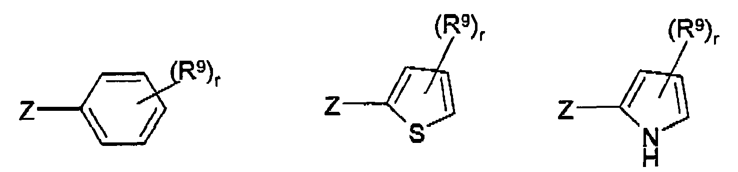 worin
R⁹ unabhängig ausgewählt ist unter Hal und A,
r für 0, 1, 2, 3, 4 oder 5 steht,
sowie die pharmazeutisch verwendbaren Derivate, Salze, Solvate und Stereoisomere und Mischungen davon.

2. Verbindungen der Formel I nach Anspruch 1, ausgewählt unter Verbindungen der Formel Ia, 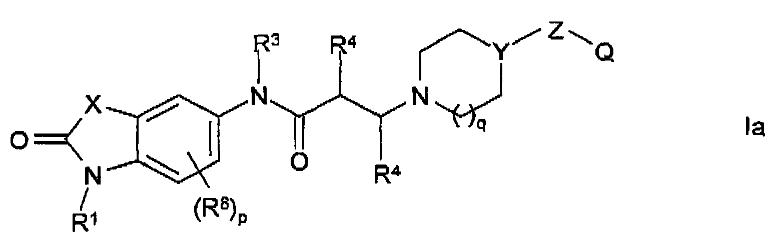 worin
q 0, 1, 2 oder 3, und
Y CH, COH oder N bedeuten,
sowie die pharmazeutisch verwendbaren Derivate, Salze, Solvate und Stereoisomere und Mischungen davon.

3. Verbindungen der Formel I nach Anspruch 1, ausgewählt unter Verbindungen der Formel Ic, 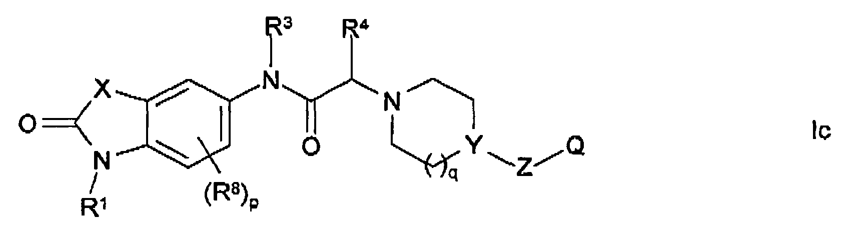 worin
R¹ H oder A
A geradkettiges oder verzweigtes Alkyl mit 1 bis 10 C-Atomen, Alkenyl mit 2 bis 10 C-Atomen, Alkoxy mit 1 bis 10 C-Atomen oder Alkoxyalkyl mit 2 bis 10 C-Atomen,
X O, S, N-R², CH₂ oder CH₂CH₂,
R² H oder A
R³ H oder A,
R⁴ H, A oder (CH₂)ₙAr,
R⁶ H, A oder Cycloalkyl mit 3 bis 7 C-Atomen,
Y CH, COH oder N,
Z O, S, N-R¹⁵, CH₂ oder CH₂CH₂,
R¹⁵ H, A, (CH₂)ₙHet, (CH₂)ₙAr oder Cycloalkyl mit 3 bis 7 C-Atomen,
Het einen unsubstituierten oder einfach oder mehrfach durch A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A und/oder OOCR⁶ substituierten, gesättigten, ungesättigten oder aromatischen mono- oder bicyclischen heterocyclischen Rest, ausgewählt unter 1-Piperidyl, 1-Piperazyl, 1-(4-Methyl)-piperazyl, 4-Methylpiperazin-1-ylamin, 4-Morpholinyl, 1-Pyrrolidinyl, 1-Pyrazolidinyl 1-(2-Methyl)-pyrazolidinyl, 1-Imidazolidinyl oder 1-(3-Methyl)-imidazolidinyl,Thiophen-2-yl oder Thiophen-3-yl, 2-, 3- oder 4-Pyridyl, das unsubstituiert oder durch eine oder mehrere CN-Gruppe substituiert sein kann, 2-, 4- oder 5-Oxazolyl, 2-, 4- oder 5-Thiazolyl, Chinolinyl, Isochinolinyl, 2- oder 4-Pyridazyl, 2-, 4- oder 5-Pyrimidyl, 2- oder 3-Pyrazinyl,
Ar einen unsubstituierten oder einfach oder mehrfach durch A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A und/oder OOCR⁶ substituierten aromatischen Kohlenwasserstofferest mit 6 bis 14 Kohlenstoffatomen,
n unabhängig voneinander 0, 1, 2, 3, 4 oder 5
p, q unabhängig voneinander 0, 1, 2 oder 3
w 0, 1, 2 oder 3
und
Hal F, Cl, Br oder I
bedeuten, und die Reste
R⁸ unabhängig voneinander ausgewählt sind unter den von H verschiedenen Bedeutungen von R⁴ oder unabhängig voneinander für Hal, CN, NO₂, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A, OOCR⁶ und/oder C(NH)NOH stehen,
sowie die pharmazeutisch verwendbaren Derivate, Salze, Solvate und Stereoisomere und Mischungen davon.

4. Verbindungen der Formel I nach Anspruch 1, ausgewählt unter Verbindungen der Formel Ie, 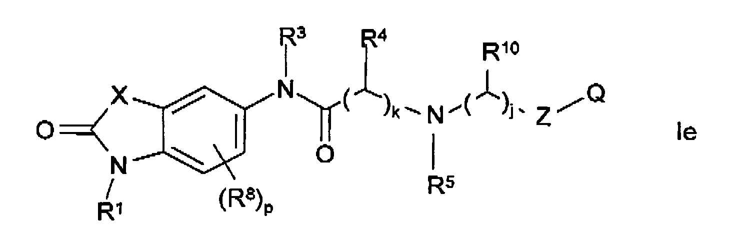 worin
R⁵ H oder A,
R¹⁰ unabhängig H, A oder (CH₂)ₙAr,
und
j 2 oder 3 bedeutet,
sowie die pharmazeutisch verwendbaren Derivate, Salze, Solvate und Stereoisomere und Mischungen davon.

5. Verbindungen nach einem der Ansprüche 1 bis 4, worin Z-Q für 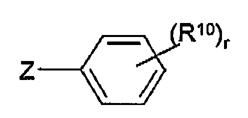 steht,
worin die Reste
R⁹ unabhängig ausgewählt sind unter Hal und A, und
r für 0, 1, 2, 3, 4 oder 5 steht.

6. Verbindungen nach Anspruch 1, ausgewählt unter
a) 3-[4-(3,4-Methylendioxybenzyl)-piperidino]-N-(2-oxo-2,3-dihydrobenzoxazol-6-yl)-propionamid
b) 3-[4-(4-Methylbenzyl)-piperidino]-N-(2-oxo-2,3-dihydrobenzoxazol-6-yl)-propionamid
c) 3-[4-(4-Methoxybenzyl)-1-piperidyl]-N-(2-oxo-2,3-dihydrobenzoxazol-6-yl)-propionamid
d) 5-Chlor-6-[3-(4-(4-fluorbenzyl)-1-piperidyl)-propionamido]-2,3-dihydrobenzoxazol-2-on
e) 3-[4-(4-Fluorbenzyl)-1-piperidyl]-N-(2-oxo-2,3-dihydro-benzoxazol-6-yl)-propionamid
f) N-(2,3-Dihydro-2-oxo-6-benzoxazolyl)-3-[4-(4-fluorbenzyl)-1-piperidyl]-propionamid
g) 2-[4-(4-Fluor-benzyl)-4-hydroxy-piperidin-1-yl]-N-(2-oxo-2,3-dihydro-benzooxazol-6-yl)-acetamid;
h) 2-[4-(4-Fluor-phenoxy)-piperidin-1-yl]-N-(2-oxo-2,3-dihydrobenzooxazol-6-yl)-propionamid;
i) 2-[4-(4-Fluor-benzyl)-piperidin-1-yl]-N-(2-oxo-2,3-dihydrobenzooxazol-6-yl)-propionamid;
j) 2-(4-Benzyl-piperazin-1-yl)-N-(2-oxo-2,3-dihydro-benzooxazol-6-yl)-propionamid;
k) 2-[4-(4-Fluor-benzyl)-4-hydroxy-piperidin-1-yl]-N-(2-oxo-2,3-dihydro-benzooxazol-6-yl)-propionamid;
l) 2-[4-(3,5-Difluor-benzyl)-4-hydroxy-piperidin-1-yl]-N-(2-oxo-2,3-dihydro-benzooxazol-6-yl)-propionamid;
m) 6-(2-(4-(4-Fluorbenzyl)-1-piperidyl)-acetamido)-2,3-dihydrobenzoxazol-2-on;
n) 2-[4-(4-Fluor-benzyl)-piperidin-1-yl]-N-(2-oxo-2,3-dihydrobenzooxazol-6-yl)-2-phenyl-acetamid;
o) 2-[4-(4-Fluor-phenoxy)-piperidin-1-yl]-N-(2-oxo-2,3-dihydrobenzooxazol-6-yl)-2-phenyl-acetamid;
p) 2-[4-(2,4-Difluor-benzyl)-piperidin-1-yl]-N-(2-oxo-2,3-dihydrobenzooxazol-6-yl)-propionamid;
q) 2-(4-Benzyl-1-piperidyl)-N-(2-oxo-2,3-dihydrobenzoxazol-6-yl)-acetamid;
r) N-(2-Oxo-2,3-dihydrobenzoxazol-6-yl)-2-(4-thiophen-2-ylmethyl-1-piperidyl)-acetamid;
s) N-(2,3-Dihydro-2-oxobenzoxazol-6-yl)-2-[4-(4-fluorphenyl)-butylamino]-acetamid
t) N-(2,3-Dihydrobenzoxazol-6-yl)-2-{N-[4-(4-fluorphenyl)-butyl]-N-methylamino}-acetamid;
sowie die pharmazeutisch verwendbaren Derivate, Salze, Solvate und Stereoisomere und Mischungen davon.

7. Verfahren zur Herstellung von Verbindungen der Formel I nach Anspruch 1 sowie ihrer Salze, **dadurch gekennzeichnet, dass** man
a) eine Verbindung der Formel II 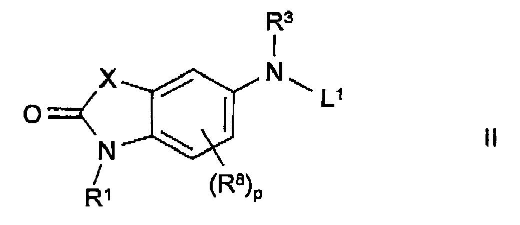 worin
L¹ H oder ein Metallion bedeutet,
und R¹, X, R³, R⁸ und p die in Anspruch 1 angegebenen Bedeutungen haben,
b) mit einer Verbindung der Formel V 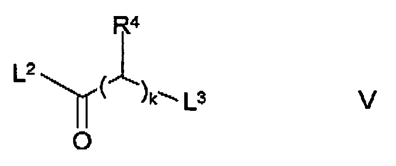 worin
L² Cl, Br, I, OH oder eine reaktionsfähig veresterte OH-Gruppe bedeutet,
L³ Cl, Br, I, OH oder eine Diazoniumgruppe bedeutet und R⁴ und k die in Anspruch 1 angegebene Bedeutung haben,
und
c) mit einer Verbindung der Formel VI 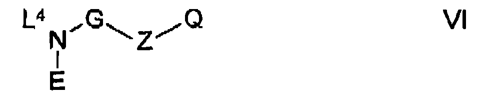 worin
L⁴ H oder ein Metallion bedeutet und E, G, Z, und Q die in Anspruch 1 angegebenen Bedeutungen haben,
umsetzt, und gegebenenfalls
d) die erhaltene Verbindung der Formel I durch Behandeln mit einer Säure in eines ihrer Salze überführt.

8. Verfahren zur Herstellung einer pharmazeutischen Zubereitung, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

9. Pharmazeutische Zubereitung, **gekennzeichnet durch** einen wirksamen Gehalt an mindestens einer Verbindung der Formel I nach Anspruch 1 und/oder einem ihrer physiologisch unbedenklichen Salze.

10. Verbindungen der Formel I nach Anspruch 1 und/oder ihre physiologisch unbedenklichen Salze als exzitatorische Aminosäuren-Antagonisten.

11. Verbindungen der Formel I nach Anspruch 1 und ihre physiologisch unbedenklichen Salze als exzitatorische Aminosäuren-Antagonisten zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen.

12. Verwendung der Verbindungen der Formel I nach Anspruch 1 und/oder ihre physiologisch unbedenklichen Salze zur Herstellung eines Arzneimittels zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen.

13. Verbindungen der Formel I nach Anspruch 1 und ihre physiologisch unbedenklichen Salze und Solvate als Arzneimittel.

14. Verbindungen der Formel I nach Anspruch 1 und ihre physiologisch unbedenklichen Salze und Solvate als Glycin-Transporter-Inhibitor.

15. Pharmazeutische Zubereitung, **gekennzeichnet durch** einen Gehalt an mindestens einer Verbindung der Formel I nach Anspruch 1 und/oder einem ihrer physiologisch unbedenklichen Salze und/oder eines ihrer Solvate.

16. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze und/oder eines ihrer Solvate zusammen mit mindestens einem festen, flüssigen oder halbflüssigen Träger- oder Hilfsstoff in eine geeignete Dosierungsform bringt.

17. Verwendung von Verbindungen der Formel I nach Anspruch 1 und/oder ihre physiologisch unbedenklichen Salze oder Solvate zur Herstellung eines Arzneimittels zur Prophylaxe und/oder Behandlung von Schizophrenie, Depression, Demenz, Parkinsonschen Krankheit, Morbus Alzheimer, Lewy Bodies Dementia, Huntington, Tourette Syndrom, Angst, Lern- und Erinnerungseinschränkungen, neurodegenerativen Erkrankungen und anderen kognitiven Beeinträchtigungen, sowie Nikotinabhängigkeit und Schmerzen.

18. Verwendung von Verbindungen der Formel II, 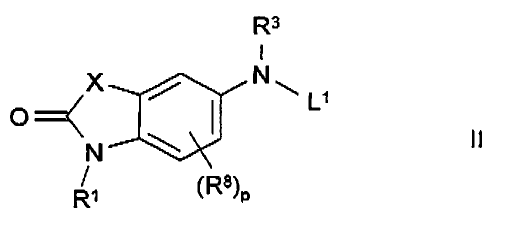 worin
L¹ H oder ein Metallion bedeutet,
und R¹, X, R³, R⁸ und p die Anspruch 7 angegebenen Bedeutungen haben,
zur Herstellung von Verbindungen der Formel I gemäß Anspruch 1 pharmazeutisch verwendbaren Derivaten, Salzen, Solvaten, Stereoisomeren und/oder Mischungen davon.

19. Verwendung von Verbindungen der Formel VI, 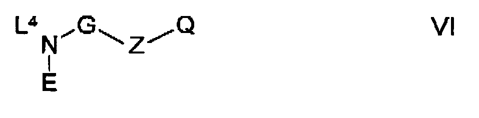 worin
L⁴ H oder ein Metallion bedeutet und E, G, Z, und Q die Anspruch 7 angegebenen Bedeutungen haben,
zur Herstellung von Verbindungen der Formel I gemäß Anspruch 1 pharmazeutisch verwendbaren Derivaten, Salzen, Solvaten, Stereoisomeren und/oder Mischungen davon.

## Claims

1. Compounds of the formula I 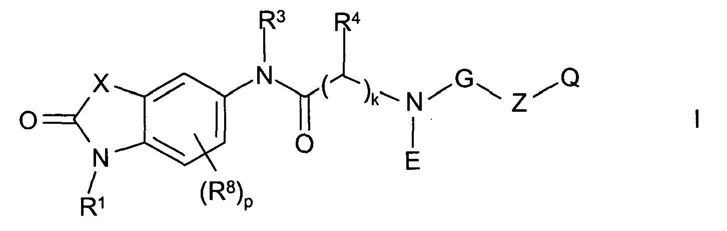 in which
R¹ denotes H or A
A denotes straight-chain or branched alkyl having 1 to 10 C atoms, alkenyl having 2 to 10 C atoms, alkoxy having 1 to 10 C atoms or alkoxyalkyl having 2 to 10 C atoms,
X denotes O, S, N-R², CH₂ or CH₂CH₂,
R² denotes H or A and
R³ denotes H or A,
R⁴ independently is selected from H, A or (CH₂)ₙAr,
R⁶ stands for H, A or cycloalkyl having 3 to 7 C atoms, and
R⁸ stands for A, Hal, CN, NO₂, NH₂, CF₃, OCF₃ or SO₂CH₃, and
k stands for 1 or 2,
the group 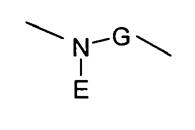 is selected from the groups 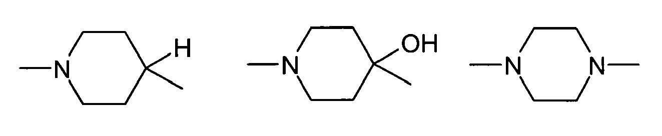 and 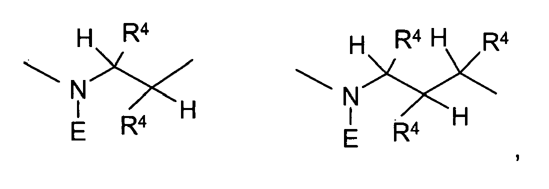
E denotes H, A, (CH₂)ₙHet, (CH₂)ₙAr or cycloalkyl having 3 to 7 C atoms,
Z denotes O, S, N-R¹⁵, CH₂ or CH₂CH₂,
R¹⁵ denotes H, A, (CH₂)ₙHet, (CH₂)ₙAr or cycloalkyl having 3 to 7 C atoms,
Het denotes a saturated, unsaturated or aromatic mono- or bicyclic heterocyclic radical selected from 1-piperidyl, 1-piperazyl, 1-(4-methyl)piperazyl, 4-methylpiperazin-1-ylamine, 4-morpholinyl, 1-pyrrolidinyl, 1-pyrazolidinyl, 1-(2-methyl)pyrazolidinyl, 1-imidazolidinyl or 1-(3-methyl)imidazolidinyl, thiophen-2-yl or thiophen-3-yl, 2-, 3- or 4-pyridyl, which may be unsubstituted or substituted by one or more CN groups, 2-, 4- or 5-oxazolyl, 2-, 4- or 5-thiazolyl, quinolinyl, isoquinolinyl, 2- or 4-pyridazyl, 2-, 4- or 5-pyrimidyl, 2- or 3-pyrazinyl which is unsubstituted or mono- or polysubstituted by A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A and/or OOCR⁶,
Ar denotes an aromatic hydrocarbon radical having 6 to 14 carbon atoms which is unsubstituted or mono- or polysubstituted by A, Hal, NO₂, CN, OR⁶, N(R⁶)₂, COOR⁶, CON(R⁶)_{2,} NR⁶COR⁶, NR⁶CON(R⁶)₂, NR⁶SO₂A, COR⁶, SO₂NR⁶, S(O)_{w}A and/or OOCR⁶,
n denotes 0, 1, 2, 3, 4 or 5,
p denotes 0, 1, 2 or 3,
w denotes 0, 1, 2 or 3,
and
Hal denotes F, Cl, Br or I,
and the group
Z-Q is selected from the groups 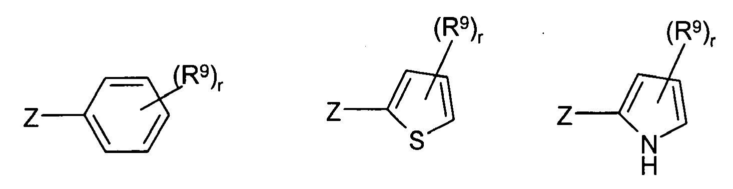 in which
R⁹ independently is selected from Hal and A,
r stands for 0, 1, 2, 3, 4 or 5,
and the pharmaceutically usable derivatives, salts, solvates and stereoisomers and mixtures thereof.

2. Compounds of the formula I according to Claim 1, selected from compounds of the formula la 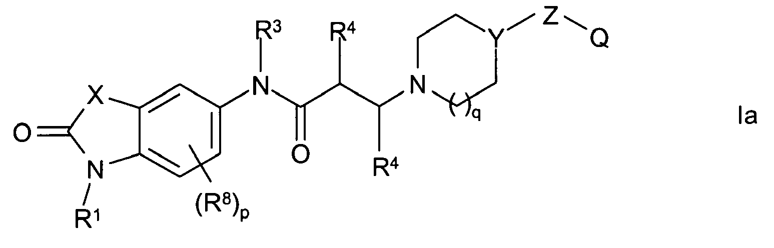 in which
q denotes 0, 1, 2 or 3, and
Y denotes CH, COH or N,
and the pharmaceutically usable derivatives, salts, solvates and stereoisomers and mixtures thereof.

3. Compounds of the formula I according to Claim 1, selected from compounds of the formula Ic 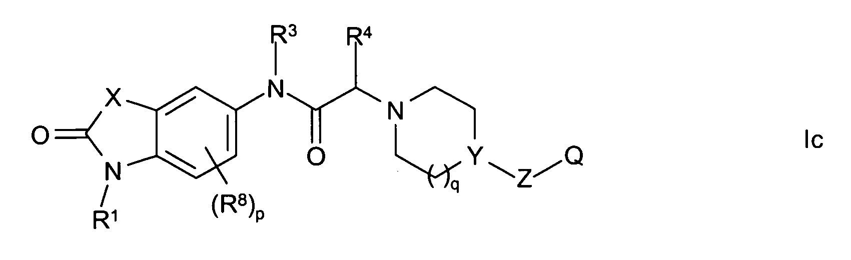 in which
R¹ denotes H or A
A denotes straight-chain or branched alkyl having 1 to 10 C atoms, alkenyl having 2 to 10 C atoms, alkoxy having 1 to 10 C atoms or alkoxyalkyl having 2 to 10 C atoms,
X denotes O, S, N-R², CH₂ or CH₂CH₂,
R² denotes H or A
R³ denotes H or A,
R⁴ denotes H, A or (CH₂)ₙAr,
R⁶ denotes H, A or cycloalkyl having 3 to 7 C atoms,
Y denotes CH, COH or N,
Z denotes O, S, N-R¹⁵, CH₂ or CH₂CH₂,
R¹⁵ denotes H, A, (CH₂)ₙHet, (CH₂)ₙAr or cycloalkyl having 3 to 7 C atoms,
Het denotes a saturated, unsaturated or aromatic mono- or bicyclic heterocyclic radical selected from 1-piperidyl, 1-piperazyl, 1-(4-methyl)piperazyl, 4-methylpiperazin-1-ylamine, 4-morpholinyl, 1-pyrrolidinyl, 1-pyrazolidinyl, 1-(2-methyl)pyrazolidinyl, 1-imidazolidinyl or 1-(3-methyl)imidazolidinyl, thiophen-2-yl or thiophen-3-yl, 2-, 3- or 4-pyridyl, which may be unsubstituted or substituted by one or more CN groups, 2-, 4- or 5-oxazolyl, 2-, 4- or 5-thiazolyl, quinolinyl, isoquinolinyl, 2- or 4-pyridazyl, 2-, 4- or 5-pyrimidyl, 2- or 3-pyrazinyl which is unsubstituted or mono- or polysubstituted by A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A and/or OOCR⁶,
Ar denotes an aromatic hydrocarbon radical having 6 to 14 carbon atoms which is unsubstituted or mono- or polysubstituted by A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A and/or OOCR⁶,
n, independently of one another, denotes 0, 1, 2, 3, 4 or 5
p, q, independently of one another, denote 0, 1, 2 or 3
w denotes 0, 1, 2 or 3
and
Hal denotes F, Cl, Br or I,
and the radicals
R⁸ are selected, independently of one another, from the meanings of R⁴ other than H or, independently of one another, stands for Hal, CN, NO₂, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A, OOCR⁶ and/or C(NH)NOH,
and the pharmaceutically usable derivatives, salts, solvates and stereoisomers and mixtures thereof.

4. Compounds of the formula I according to Claim 1, selected from compounds of the formula Ie 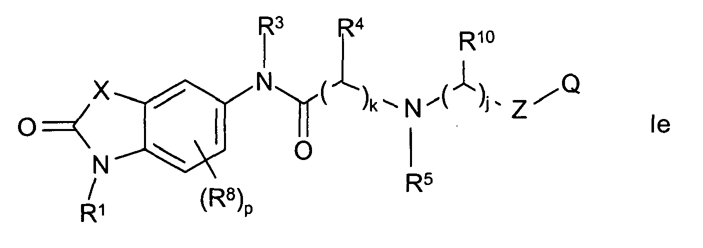 in which
R⁵ denotes H or A,
R¹⁰ independently denotes H, A or (CH₂)ₙAr,
and
j denotes 2 or 3,
and the pharmaceutically usable derivatives, salts, solvates and stereoisomers and mixtures thereof.

5. Compounds according to one of Claims 1 to 4, in which Z-Q stands for 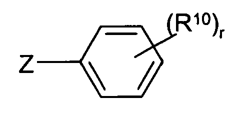 in which the radicals
R⁹ independently are selected from Hal and A, and
r stands for 0, 1, 2, 3, 4 or 5.

6. Compounds according to Claim 1, selected from
a) 3-[4-(3,4-methylenedioxybenzyl)piperidino]-N-(2-oxo-2,3-dihydrobenzoxazol-6-yl)propionamide
b) 3-[4-(4-methylbenzyl)piperidino]-N-(2-oxo-2,3-dihydrobenzoxazol-6-yl)propionamide
c) 3-[4-(4-methoxybenzyl)-1-piperidyl]-N-(2-oxo-2,3-dihydrobenzoxazol-6-yl)propionamide
d) 5-chloro-6-[3-(4-(4-fluorobenzyl)-1-piperidyl)propionamido]-2,3-dihydrobenzoxazol-2-one
e) 3-[4-(4-fluorobenzyl)-1-piperidyl]-N-(2-oxo-2,3-dihydrobenzoxazol-6-yl)propionamide
f) N-(2,3-dihydro-2-oxo-6-benzoxazolyl)-3-[4-(4-fluorobenzyl)-1-piperidyl]propionamide
g) 2-[4-(4-fluorobenzyl)-4-hydroxypiperidin-1-yl]-N-(2-oxo-2,3-dihydrobenzoxazol-6-yl)acetamide;
h) 2-[4-(4-fluorophenoxy)piperidin-1-yl]-N-(2-oxo-2,3-dihydrobenzoxazol-6-yl)propionamide;
i) 2-[4-(4-fluorobenzyl)piperidin-1-yl]-N-(2-oxo-2,3-dihydrobenzoxazol-6-yl)propionamide;
j) 2-(4-benzylpiperazin-1-yl)-N-(2-oxo-2,3-dihydrobenzoxazol-6-yl)-propionamide;
k) 2-[4-(4-fluorobenzyl)-4-hydroxypiperidin-1-yl]-N-(2-oxo-2,3-dihydrobenzoxazol-6-yl)propionamide;
l) 2-[4-(3,5-difluorobenzyl)-4-hydroxypiperidin-1-yl]-N-(2-oxo-2,3-dihydrobenzoxazol-6-yl)propionamide;
m) 6-(2-(4-(4-fluorobenzyl)-1-piperidyl)acetamido)-2,3-dihydrobenzoxazol-2-one;
n) 2-[4-(4-fluorobenzyl)piperidin-1-yl]-N-(2-oxo-2,3-dihydrobenzoxazol-6-yl)-2-phenylacetamide;
o) 2-[4-(4-fluorophenoxy)piperidin-1-yl]-N-(2-oxo-2,3-dihydrobenzoxazol-6-yl)-2-phenylacetamide;
p) 2-[4-(2,4-difluorobenzyl)piperidin-1-yl]-N-(2-oxo-2,3-dihydrobenzoxazol-6-yl)propionamide;
q) 2-(4-benzyl-1-piperidyl)-N-(2-oxo-2,3-dihydrobenzoxazol-6-yl)acetamide;
r) N-(2-oxo-2,3-dihydrobenzoxazol-6-yl)-2-(4-thiophen-2-ylmethyl-1-piperidyl)acetamide;
s) N-(2,3-dihydro-2-oxobenzoxazol-6-yl)-2-[4-(4-fluorophenyl)butylamino]acetamide
t) N-(2,3-dihydrobenzoxazol-6-yl)-2-{N-[4-(4-fluorophenyl)butyl]-N-methylamino}acetamide;
and the pharmaceutically usable derivatives, salts, solvates and stereoisomers and mixtures thereof.

7. Process for the preparation of compounds of the formula I according to Claim 1 and salts thereof, **characterised in that**
a) a compound of the formula II 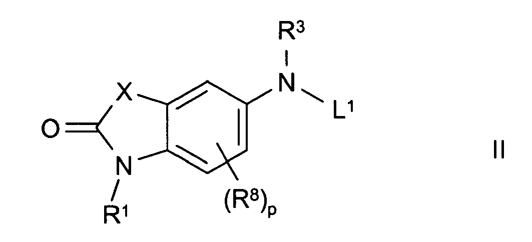 in which
L¹ denotes H or a metal ion,
and R¹, X, R³, R⁸ and p have the meanings indicated in Claim 1,
b) is reacted with a compound of the formula V 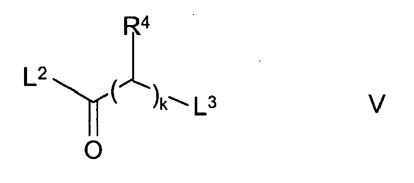 in which
L² denotes Cl, Br, I, OH or a reactively esterified OH group,
L³ denotes Cl, Br, I, OH or a diazonium group
and R⁴ and k have the meaning indicated in Claim 1,
and
c) with a compound of the formula VI 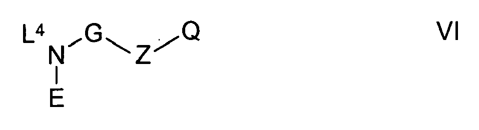 in which
L⁴ denotes H or a metal ion and E, G, Z, and Q have the meanings indicated in Claim 1,
and, if desired,
d) the resultant compound of the formula I is converted into one of its salts by treatment with an acid.

8. Process for the preparation of a pharmaceutical composition, **characterised in that** a compound of the formula I according to Claim 1 and/or one of its physiologically acceptable salts is converted into a suitable dosage form together with at least one solid, liquid or semi-liquid excipient or adjuvant.

9. Pharmaceutical composition, **characterised by** an effective content of at least one compound of the formula I according to Claim 1 and/or one of its physiologically acceptable salts.

10. Compounds of the formula I according to Claim 1 and/or physiologically acceptable salts thereof as excitatory amino acid antagonists.

11. Compounds of the formula I according to Claim 1 and physiologically acceptable salts thereof as excitatory amino acid antagonists for combating neurodegenerative diseases, including cerebrovascular diseases, epilepsy, schizophrenia, Alzheimer's disease, Parkinson's disease or Huntington's disease, cerebral ischaemia, infarction or psychoses.

12. Use of the compounds of the formula I according to Claim 1 and/or physiologically acceptable salts thereof for the preparation of a medicament for combating neurodegenerative diseases, including cerebrovascular diseases, epilepsy, schizophrenia, Alzheimer's disease, Parkinson's disease or Huntington's disease, cerebral ischaemia, infarction or psychoses.

13. Compounds of the formula I according to Claim 1 and physiologically acceptable salts and solvates thereof as medicaments.

14. Compounds of the formula I according to Claim 1 and physiologically acceptable salts and solvates thereof as glycine transporter inhibitor.

15. Pharmaceutical composition, **characterised by** a content of at least one compound of the formula I according to Claim 1 and/or one of its physiologically acceptable salts and/or one of its solvates.

16. Process for the preparation of pharmaceutical compositions, **characterised in that** a compound of the formula I according to Claim 1 and/or one of its physiologically acceptable salts and/or one of its solvates is converted into a suitable dosage form together with at least one solid, liquid or semi-liquid excipient or adjuvant.

17. Use of compounds of the formula I according to Claim 1 and/or physiologically acceptable salts or solvates thereof for the preparation of a medicament for the prophylaxis and/or treatment of schizophrenia, depression, dementia, Parkinson's disease, Alzheimer's disease, Lewy bodies dementia, Huntington's disease, Tourette's syndrome, anxiety, learning and memory impairment, neurodegenerative diseases and other cognitive impairment, as well as nicotine dependence and pain.

18. Use of compounds of the formula II 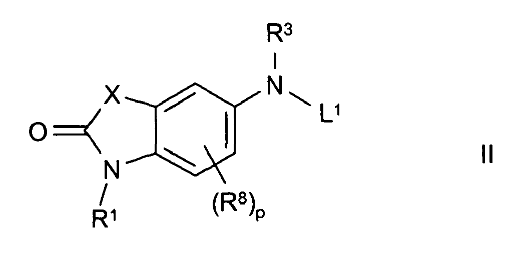 in which
L¹ denotes H or a metal ion,
and R¹, X, R³, R⁸ and p have the meanings indicated in Claim 7,
for the preparation of compounds of the formula I according to Claim 1, pharmaceutically usable derivatives, salts, solvates, stereoisomers and/or mixtures thereof.

19. Use of compounds of the formula VI 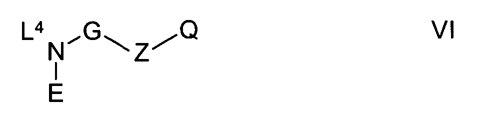 in which
L⁴ denotes H or a metal ion, and E, G, Z, and Q have the meanings indicated in Claim 7,
for the preparation of compounds of the formula I according to Claim 1, pharmaceutically usable derivatives, salts, solvates, stereoisomers and/or mixtures thereof.

## Revendications

1. Composés de formule I 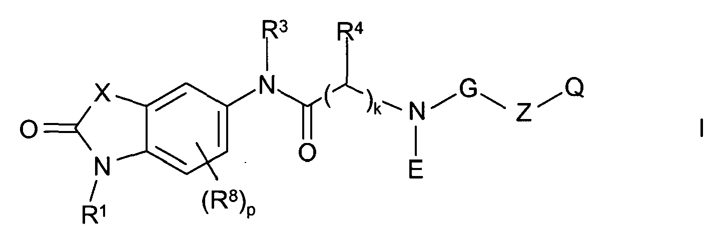 dans laquelle
R¹ désigne H ou A
A désigne alkyle ayant de 1 à 10 atomes de C, alcényle ayant de 2 à 10 atomes de C, alcoxy ayant de 1 à 10 atomes de C ou alcoxyalkyle ayant de 2 à 10 atomes de C, à chaîne linéaire ou ramifiée,
X désigne O, S, N-R², CH₂ ou CH₂CH₂,
R² désigne H ou A et
R³ désigne H ou A,
R⁴ est sélectionné indépendamment parmi H, A ou (CH₂)ₙAr,
R⁶ signifie H, A ou cycloalkyle ayant de 3 à 7 atomes de C, et
R⁸ signifie A, Hal, CN, NO₂, NH₂, CF₃, OCF₃ ou SO₂CH₃, et
k signifie 1 ou 2,
le groupement 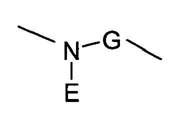 est sélectionné parmi les groupements 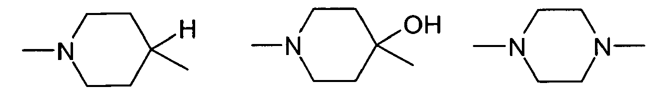 et 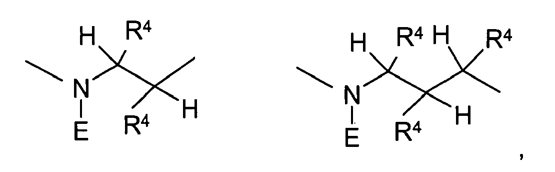
E désigne H, A, (CH₂)ₙHét, (CH₂)ₙAr ou cycloalkyle ayant de 3 à 7 atomes de C,
Z désigne O, S, N-R¹⁵, CH₂ ou CH₂CH₂,
R¹⁵ désigne H, A, (CH₂)ₙHét, (CH₂)ₙAr ou cycloalkyle ayant de 3 à 7 atomes de C,
Hét désigne un radical hétérocyclique mono- ou bicyclique, saturé, insaturé ou aromatique, sélectionné parmi 1-pipéridyle, 1-pipérazyle, 1-(4-méthyl)pipérazyle, 4-méthylpipérazin-1-ylamine, 4-morpholinyle, 1-pyrrolidinyle, 1-pyrazolidinyle, 1-(2-méthyl)pyrazolidinyle, 1-imidazolidinyle ou 1-(3-méthyl)imidazolidinyle, thiophén-2-yle ou thiophén-3-yle, 2-, 3- ou 4-pyridyle, pouvant être non substitué ou substitué par un ou plusieurs groupements CN, 2-, 4- ou 5-oxazolyle, 2-, 4- ou 5-thiazolyle, quinoléinyle, isoquinoléinyle, 2- ou 4-pyridazyle, 2-, 4- ou 5-pyrimidyle, 2- ou 3-pyrazinyle qui est non substitué ou mono- ou polysubstitué par A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A et/ou OOCR⁶,
Ar désigne un radical hydrocarboné aromatique ayant de 6 à 14 atomes de carbone, qui est non substitué ou mono- ou poly-substitué par A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A et/ou OOCR⁶,
n désigne 0, 1, 2, 3, 4 ou 5,
p désigne 0, 1, 2 ou 3,
w désigne 0, 1, 2 ou 3,
et
Hal désigne F, Cl, Br ou I,
et le groupement
Z-Q est sélectionné parmi les groupements 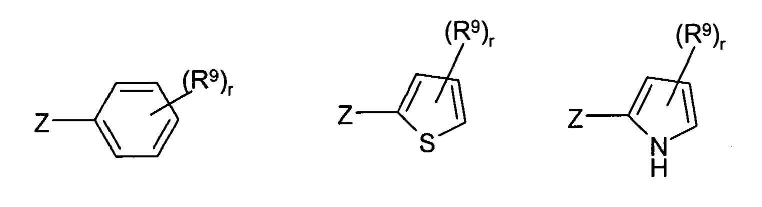 dans lesquels
R⁹ est sélectionné indépendamment parmi Hal et A,
r signifie 0, 1, 2, 3, 4 ou 5,
et les dérivés, sels, solvats et stéréoisomères pharmaceutiquement utilisables et mélanges de ceux-ci.

2. Composés de formule I selon la revendication 1, sélectionnés parmi les composés de formule la 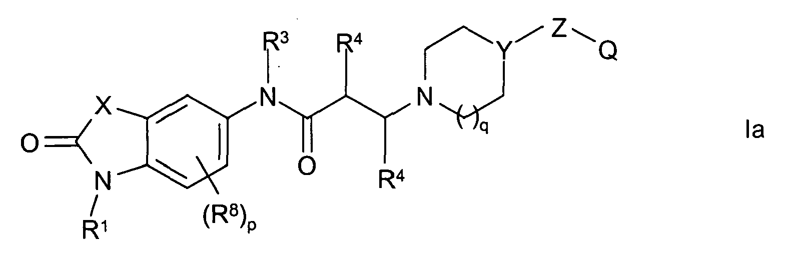 dans laquelle
q désigne 0, 1, 2 ou 3, et
Y désigne CH, COH ou N,
et les dérivés, sels, solvats et stéréoisomères pharmaceutiquement utilisables et mélanges de ceux-ci.

3. Composés de formule I selon la revendication 1, sélectionnés parmi les composés de formule Ic 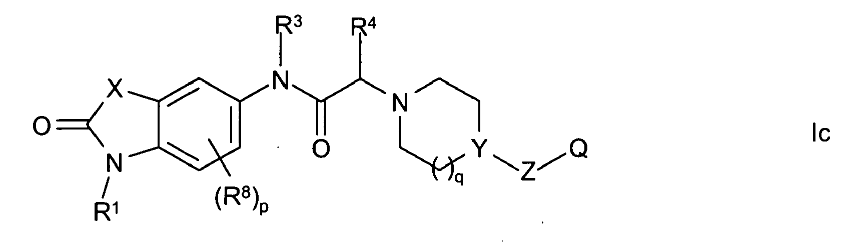 dans laquelle
R¹ désigne H ou A
A désigne alkyle ayant de 1 à 10 atomes de C, alcényle ayant de 2 à 10 atomes de C, alcoxy ayant de 1 à 10 atomes de C ou alcoxyalkyle ayant de 2 à 10 atomes de C, à chaîne linéaire ou ramifiée,
X désigne O, S, N-R², CH₂ ou CH₂CH₂,
R² désigne H ou A
R³ désigne H ou A,
R⁴ désigne H, A ou (CH₂)ₙAr,
R⁶ désigne H, A ou cycloalkyle ayant de 3 à 7 atomes de C,
Y désigne CH, COH ou N,
Z désigne O, S, N-R¹⁵, CH2 ou CH₂CH₂,
R¹⁵ désigne H, A, (CH₂)ₙHét, (CH₂)ₙAr ou cycloalkyle ayant de 3 à 7 atomes de C,
Hét désigne un radical hétérocyclique mono- ou bicyclique, saturé, insaturé ou aromatique, sélectionné parmi 1-pipéridyle, 1-pipérazyle, 1-(4-méthyl)pipérazyle, 4-méthylpipérazin-1-ylamine, 4-morpholinyle, 1-pyrrolidinyle, 1-pyrazolidinyle, 1-(2-méthyl)pyrazolidinyle, 1-imidazolidinyle ou 1-(3-méthyl)imidazolidinyle, thiophén-2-yle ou thiophén-3-yle, 2-, 3- ou 4-pyridyle, pouvant être non substitué ou substitué par un ou plusieurs groupements CN, 2-, 4- ou 5-oxazolyle, 2-, 4- ou 5-thiazolyle, quinoléinyle, isoquinoléinyle, 2- ou 4-pyridazyle, 2-, 4- ou 5-pyrimidyle, 2- ou 3-pyrazinyle qui est non substitué ou mono- ou polysubstitué par A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A et/ou OOCR⁶,
Ar désigne un radical hydrocarboné aromatique ayant de 6 à 14 atomes de carbone, qui est non substitué ou mono- ou poly-substitué par A, Hal, NO₂, CN, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A et/ou OOCR⁶,
n, indépendamment les uns des autres, désigne 0, 1, 2, 3, 4 ou 5
p, q, indépendamment les uns des autres, désignent 0, 1, 2 ou 3
w désigne 0, 1, 2 ou 3
et
Hal désigne F, Cl, Br ou I,
et les radicaux
R⁸ sont sélectionnés, indépendamment les uns des autres, parmi les significations de R⁴ autres que H ou, indépendamment les uns des autres, signifie Hal, CN, NO₂, OR⁶, N(R⁶)₂, COOR⁶, CON(R⁶)₂, NR⁶COR⁶, NR⁶CON(R⁶)₂, NR⁶SO₂A, COR⁶, SO₂NR⁶, S(O)_{w}A, OOCR⁶ et/ou C(NH)NOH,
et les dérivés, sels, solvats et stéréoisomères pharmaceutiquement utilisables et mélanges de ceux-ci.

4. Composés de formule I selon la revendication 1, sélectionnés parmi les composés de formule le 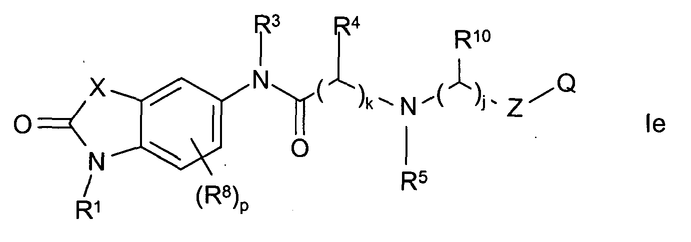 dans laquelle
R⁵ désigne H ou A,
R¹⁰ désigne indépendamment H, A ou (CH₂)ₙAr,
et
j désigne 2 ou 3,
et les dérivés, sels, solvats et stéréoisomères pharmaceutiquement utilisables et mélanges de ceux-ci.

5. Composés selon l'une des revendications 1 à 4, dans lesquels Z-Q signifie 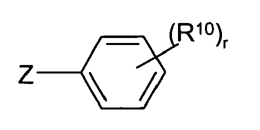 où les radicaux
R⁹ sont sélectionnés indépendamment parmi Hal et A, et
r signifie 0, 1, 2, 3, 4 ou 5.

6. Composés selon la revendication 1, sélectionnés parmi
a) le 3-[4-(3,4-méthylènedioxybenzyl)pipéridino]-N-(2-oxo-2,3-dihydrobenzoxazol-6-yl)propionamide ;
b) le 3-[4-(4-méthylbenzyl)pipéridino]-N-(2-oxo-2,3-dihydrobenzoxazol-6-yl)propionamide ;
c) le 3-[4-(4-méthoxybenzyl)-1-pipéridyl]-N-(2-oxo-2,3-dihydrobenzoxazol-6-yl)propionamide ;
d) la 5-chloro-6-[3-(4-(4-fluorobenzyl)-1-pipéridyl)propionamido]-2,3-dihydrobenzoxazol-2-one ;
e) le 3-[4-(4-fluorobenzyl)-1-pipéridyl]-N-(2-oxo-2,3-dihydrobenzoxazol-6-yl)propionamide ;
f) le N-(2,3-dihydro-2-oxo-6-benzoxazolyl)-3-[4-(4-fluorobenzyl)-1-pipéridyl]propionamide ;
g) le 2-[4-(4-fluorobenzyl)-4-hydroxypipéridin-1-yl]-N-(2-oxo-2,3-dihydrobenzoxazol-6-yl)acétamide ;
h) le 2-[4-(4-fluorophénoxy)pipéridin-1-yl]-N-(2-oxo-2,3-dihydrobenzoxazol-6-yl)propionamide ;
i) le 2-[4-(4-fluorobenzyl)pipéridin-1-yl]-N- (2-oxo-2,3-dihydrobenzoxazol-6-yl)propionamide ;
j) le 2-(4-benzylpipérazin-1-yl)-N-(2-oxo-2,3-dihydrobenzoxazol-6-yl)propionamide ;
k) le 2-[4-(4-fluorobenzyl)-4-hydroxypipéridin-1-yl]-N-(2-oxo-2,3-dihydrobenzoxazol-6-yl)propionamide ;
l) le 2-[4-(3,5-difluorobenzyl)-4-hydroxypipéridin-1-yl]-N-(2-oxo-2,3-dihydrobenzoxazol-6-yl)propionamide ;
m) la 6-(2-(4-(4-fluorobenzyl)-1-pipéridyl)acétamido)-2,3-dihydrobenzoxazol-2-one ;
n) le 2-[4-(4-fluorobenzyl)pipéridin-1-yl]-N-(2-oxo-2,3-dihydrobenzoxazol-6-yl)-2-phénylacétamide ;
o) le 2-[4-(4-fluorophénoxy)pipéridin-1-yl]-N-(2-oxo-2,3-dihydrobenzoxazol-6-yl)-2-phénylacétamide ;
p) le 2-[4-(2,4-difluorobenzyl)pipéridin-1-yl]-N-(2-oxo-2,3-dihydrobenzoxazol-6-yl)propionamide ;
q) le 2-(4-benzyl-1-pipéridyl)-N-(2-oxo-2,3-dihydrobenzoxazol-6-yl)-acétamide ;
r) le N-(2-oxo-2,3-dihydrobenzoxazol-6-yl)-2-(4-thiophén-2-ylméthyl-1-pipéridyl)acétamide ;
s) le N-(2,3-dihydro-2-oxobenzoxazol-6-yl)-2-[4-(4-fluorophényl)butylamino]acétamide
t) le N-(2,3-dihydrobenzoxazol-6-yl)-2-{N-[4-(4-fluorophényl)butyl]-N-méthylamino}acétamide ;
et les dérivés, sels, solvats et stéréoisomères pharmaceutiquement utilisables et mélanges de ceux-ci.

7. Procédé de préparation des composés de formule I selon la revendication 1 et de sels de ceux-ci, **caractérisé en ce que**
a) un composé de formule II 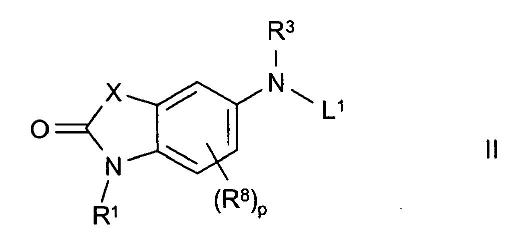 dans laquelle
L¹ désigne H ou un ion métallique,
et R¹, X, R³, R⁸ et p ont les significations indiquées selon la revendication 1,
b) est réagi avec un composé de formule V 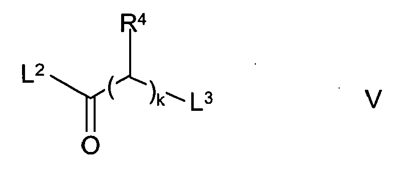 dans laquelle
L² désigne Cl, Br, I, OH ou un groupement OH estérifié de manière réactive,
L³ désigne Cl, Br, I, OH ou un groupement diazonium et R⁴ et k ont la signification indiquée selon la revendication 1,
et
c) avec un composé de formule VI 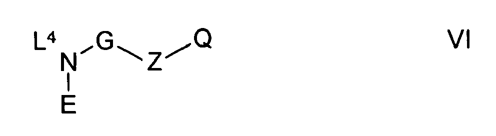 dans laquelle
L⁴ désigne H ou un ion métallique et E, G, Z, et Q ont les significations indiquées selon la revendication 1,
et, si on le souhaite,
d) le composé résultant de formule I est converti en l'un de ses sels par traitement à l'aide d'un acide.

8. Procédé de préparation d'une composition pharmaceutique, **caractérisé en ce qu'**un composé de formule I selon la revendication 1 et/ou l'un de ses sels physiologiquement acceptables est converti en une forme de dosage convenable conjointement avec au moins un excipient ou adjuvant solide, liquide ou semi-liquide.

9. Composition pharmaceutique, **caractérisée par** une teneur efficace en au moins un composé de formule I selon la revendication 1 et/ou en l'un de ses sels physiologiquement acceptables.

10. Composés de formule I selon la revendication 1 et/ou sels physiologiquement acceptables de ceux-ci, comme antagonistes d'acides aminés excitateurs.

11. Composés de formule I selon la revendication 1 et sels physiologiquement acceptables de ceux-ci, comme antagonistes d'acides aminés excitateurs destinés à combattre des maladies neurodégénératives, y compris les maladies cérébrovasculaires, l'épilepsie, la schizophrénie, la maladie d'Alzheimer, de Parkinson ou de Huntington, l'ischémie cérébrale, les infarctus ou les psychoses.

12. Utilisation des composés de formule I selon la revendication 1 et/ou de sels physiologiquement acceptables de ceux-ci, pour la préparation d'un médicament destiné à lutter contre des maladies neurodégénératives, y compris les maladies cérébrovasculaires, l'épilepsie, la schizophrénie, la maladie d'Alzheimer, de Parkinson ou de Huntington, l'ischémie cérébrale, les infarctus ou les psychoses.

13. Composés de formule I selon la revendication 1 et sels et solvats physiologiquement acceptables de ceux-ci, comme médicaments.

14. Composés de formule I selon la revendication 1 et sels et solvats physiologiquement acceptables de ceux-ci, comme inhibiteurs de transporteur de glycine.

15. Composition pharmaceutique, **caractérisée par** une teneur en au moins un composé de formule I selon la revendication 1 et/ou en l'un de ses sels physiologiquement acceptables et/ou en l'un de ses solvats.

16. Procédé de préparation de compositions pharmaceutiques, **caractérisé en ce qu'**un composé de formule I selon la revendication 1 et/ou l'un de ses sels physiologiquement acceptables et/ou l'un de ses solvats est converti en une forme de dosage convenable conjointement avec au moins un excipient ou adjuvant solide, liquide ou semi-liquide.

17. Utilisation de composés de formule I selon la revendication 1 et/ou de sels ou de solvats physiologiquement acceptables de ceux-ci, pour la préparation d'un médicament destiné à la prophylaxie et/ou au traitement de la schizophrénie, de la dépression, de la démence, de la maladie de Parkinson, de la maladie d'Alzheimer, de la démence à corps de Lewy, de la maladie de Huntington, du syndrome de Tourette, de l'anxiété, de déficiences de l'apprentissage et de la mémoire, des maladies neurodégénératives et d'autres déficiences cognitives, ainsi que du tabagisme et des douleurs.

18. Utilisation de composés de formule II 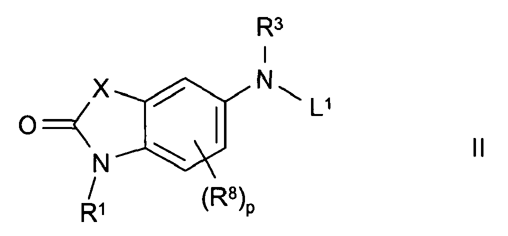 dans laquelle
L¹ désigne H ou un ion métallique,
et R¹, X, R³, R⁸ et p ont les significations indiquées selon la revendication 7,
pour la préparation de composés de formule I selon la revendication 1, de dérivés, sels, solvats ou stéréoisomères pharmaceutiquement utilisables et/ou mélanges de ceux-ci.

19. Utilisation de composés de formule VI 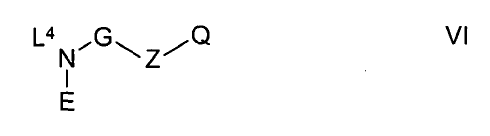 dans laquelle
L⁴ désigne H ou un ion métallique, et E, G, Z, et Q ont les significations indiquées selon la revendication 7,
pour la préparation de composés de formule I selon la revendication 1, de dérivés, sels, solvats ou stéréoisomères pharmaceutiquement utilisables et/ou mélanges de ceux-ci.
